# EUROPÄISCHE PATENTSCHRIFT

(11) **EP 0 492 366 B1**
(45) Veröffentlichungstag und Bekanntmachung des Hinweises auf die Patenterteilung: **26.03.1997**
(21) Anmeldenummer: 91121622.4
(22) Anmeldetag: 17.12.1991
(51) Int. Cl.: C07D 215/28, C07D 405/12, C07F 7/18, A01N 43/42

(54) **Neue 5-Chlorchinolin-8-oxyalkancarbonsäurederivate, Verfahren zu ihrer Herstellung und ihre Verwendung als Antidots von Herbiziden**
New 5-chloroquinolin-8-oxyalkanecarbonic acid derivatives, process for their preparation and their use as antidotes for herbicides
Nouveaux dérivés de chloro-5-quinoline-8-acide oxyalkanecarboniques, procédé pour leur préparation et leur utilisation comme antidote d'herbicides

(30) Priorität: 21.12.1990 DE 4041121
(43) Veröffentlichungstag der Anmeldung: 01.07.1992
(73) Patentinhaber: Hoechst Schering AgrEvo GmbH, 13342 Berlin (DE)
(72) Erfinder: Schütze, Rainer, Dr., W-6233 Kelkheim (Taunus) (DE); Löher, Heinz-Josef, Dr., W-6237 Liederbach (DE); Ziemer, Frank, Dr., W-6230 Frankfurt am Main (DE); Bauer, Klaus, Dr., W-6450 Hanau (DE); Bieringer, Hermann, Dr., W-6239 Eppstein/Taunus (DE)

(56) Entgegenhaltungen:
- EP-A- 0 094 349
- EP-A- 0 138 773
- EP-A- 0 159 290
- EP-A- 0 191 736
- EP-A- 0 258 184
- The Pesticide manual, 10th Edition, Ed. Clive Tomlin, Seite 226 : Nr. 155; Cloquintocet.

## Beschreibung

Die Erfindung betrifft das technische Gebiet der Pflanzenschutzmittel, speziell der Antidote oder Safener zum Schützen von Kultur-Pflanzen gegen unerwünschte Nebenwirkungen von Herbiziden.

Es ist bereits bekannt, Verbindungen aus der Reihe der Chinolinoxyalkancarbonsäurederivate als Antidote oder Safener zusammen mit Herbiziden einzusetzen (siehe z. B. EP-A-94 349 (US-A-4,902,340), EP-A-191 736 (US-4,881,966), EP-A-0159287 (US-A-4,851,031), DE-A-25 46 845, EP-A-159 290). Jedoch zeigte sich, daß die bekannten Verbindungen anwendungstechnische Nachteile haben, beispielsweise zu geringe Safener-Wirkung aufweisen oder die Wirkung der Herbizide gegen Schadpflanzen in unerwünschter Weise vermindern.

Gegenstand der Erfindung sind neue 5-Chlorchinolin-8-oxyalkancarbonsäurederivate der Formel I, 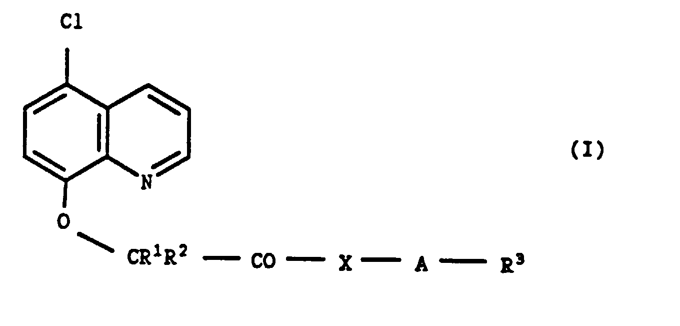 worin
- R¹,R²: unabhängig voneinander Wasserstoff oder (C₁-C₄)-Alkyl, vorzugsweise Wasserstoff oder Methyl,
- X: ein Sauerstoff- oder Schwefelatom oder NR⁴, wobei R⁴ Wasserstoff, (C₁-C₆)-Alkyl, (C₁-C₆)-Alkoxy oder gegebenenfalls substituiertes Phenyl bedeutet, vorzugsweise O, NH, oder NCH₃, insbesondere O,
- A: (C₁-C₆)-Alkylen, (C₄-C₈)-Alkenylen, (C₄-C₈)-Alkinylen, (C₃-C₈)-Cycloalkylen oder (C₃-C₈)-Cycloalkenylen,
- R³: (C₃-C₆)-Alkenyloxy, (C₃-C₆)-Alkinyloxy, Phenyl-(C₁-C₄)-alkoxy, worin der Phenylring unsubstituiert oder ein- oder mehrfach durch Reste aus der Gruppe Halogen, Nitro, (C₁-C₄)-Alkyl, (C₁-C₄)-Alkoxy, (C₁-C₄)-Haloalkyl und (C₁-C₄)-Haloalkoxy substituiert ist,
einen Rest der Formel R⁸O-CH(OR⁹)- oder R⁸O-CH(OR⁹)-(CH₂)ₙ-O-, worin n 0, 1 oder 2 bedeutet,
oder einen Alkoxy-Rest der Formel R⁸O-CHR¹⁰-CH(OR⁹)-(C₁-C₄)-alkoxy,
(C₁-C₆)-Alkylcarbonyloxy, worin Alkyl unsubstituiert oder durch Halogen, Nitro, gegebenenfalls substituiertes Phenyl oder (C₁-C₄)-Alkoxy substituiert ist,
(C₂-C₆)-Alkenylcarbonyloxy, (C₂-C₆)-Alkinylcarbonyloxy, Phenylcarbonyloxy, wobei Phenyl unsubstituiert oder ein- oder mehrfach durch Reste aus der Gruppe Halogen, Nitro, (C₁-C₄)-Alkyl, (C₁-C₄)-Alkoxy, (C₁-C₄)-Haloalkyl und (C₁-C₄)-Haloalkoxy substituiert ist,
- R⁸,R⁹: unabhängig voneinander (C₁-C₄)-Alkyl oder R⁸ und R⁹ zusammen eine geradkettige oder verzweigte (C₁-C₄)-Alkylenbrücke und
- R¹⁰: H oder (C₁-C₄)-Alkyl
bedeuten.

In den Formeln sind Alkyl, Alkenyl und Alkinyl geradkettig oder verzweigt; entsprechendes gilt für substituierte Alkyl-, Alkenyl- und Alkinylreste wie Haloalkyl, Hydroxyalkyl, Alkoxycarbonyl etc.; Alkyl bedeutet z. B. Methyl, Ethyl, n- und i-Propyl, n-, i-, t- und 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl bedeutet z. B. Allyl, 1-Methylprop-2-en-1-yl, 2-Methylprop-2-en-1-yl, But-2-en-1-yl, But-3-en-1-yl, 1-Methyl-but-3-en und 1-Methyl-but-2-en; Alkinyl bedeutet z. B. Propargyl, But-2-in-1-yl, But-3-in-1-yl, 1-Methylbut-3-in; Halogen bedeutet Fluor, Chlor, Brom oder lod, vorzugsweise Fluor, Chlor oder Brom, besonders Fluor oder Chlor; Haloalkyl, -alkenyl und -alkinyl bedeuten durch Halogen substituiertes Alkyl, Alkenyl bzw. Alkinyl, z. B. CF₃, CHF₂, CH₂F, CF₃CF₂, CH₂FCHCl, CCl₃, CHCl₂, CH₂CH₂Cl; Haloalkoxy ist z. B. OCF₃, OCHF₂, OCH₂F, CF₃CF₂O, OCH₂CF₃; gegebenenfalls substituiertes Phenyl ist z. B. Phenyl, das unsubstituiert oder ein- oder mehrfach durch Reste aus der Gruppe Halogen, (C₁-C₄)-Alkyl, (C₁-C₄)-Alkoxy, (C₁-C₄)-Halogenalkyl, (C1-C4)-Halogenalkoxy und Nitro substituiert ist, z. B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Manche Verbindungen der Formel I enthalten ein oder mehrere asymmetrische C-Atome oder Doppelbindungen, die in der allgemeinen Formel I nicht gesondert angegeben sind. Die durch ihre spezifische Raumform definierten möglichen Stereoisomeren, wie Enantiomere, Diastereomere, E- und Z-lsomere sowie deren Gemische sind jedoch alle von der Formel I umfaßt. Die reinen oder angereicherten Stereoisomeren können nach üblichen Methoden aus Gemischen der Stereoisomeren erhalten werden oder auch durch stereoselektive Reaktionen aus stereochemisch reinen Ausgangsstoffen hergestellt werden. Die genannten Stereoisomeren in reiner Form als auch ihre Gemische sind somit Gegenstand dieser Erfindung.

Von besonderem Interesse sind erfindungsgemäße Verbindungen der Formel (I), worin
- R³: (C₃-C₄)-Alkenyloxy, (C₃-C₄)-Alkinyloxy,
Phenyl-(C₁-C₂)-alkoxy, worin der Phenylring unsubstituiert oder ein- oder mehrfach durch Reste aus der Gruppe Halogen, Nitro, (C₁-C₂)-Alkyl, (C₁-C₂)-Alkoxy, (C₁-C₂)-Haloalkyl und (C₁-C₂)-Haloalkoxy substituiert ist,
(C₁-C₄)-Alkylcarbonyloxy, (C₃-C₄)-Alkenylcarbonyloxy, (C₃-C₄)-Alkinylcarbonyloxy,
Phenylcarbonyloxy, wobei Phenyl unsubstituiert oder ein- oder mehrfach durch Reste aus der Gruppe Halogen, Nitro, (C₁-C₄)-Alkyl, (C₁-C₄)-Alkoxy, (C₁-C₄)-Haloalkyl und (C₁-C₄)-Haloalkoxy substituiert ist, oder
einen Rest der Formel -O-CH₂-CH(OR')-CH₂-OR', worin die R' zusammen für die divalente Gruppe CH₂, CHCH₃ oder C(CH₃)₂ stehen, und
- R⁸,R⁹: unabhängig voneinander (C₁-C₄)-Alkyl
bedeuten.

Vorzugsweise ist
- R³: (C₃-C₄)-Alkenyloxy, (C₃-C₄)-Alkinyloxy, Benzyloxy, (C₃-C₄)-Alkenylcarbonyloxy oder (C₃-C₄)-Alkinylcarbonyloxy.

Vorzugsweise ist
- A: (C₁-C₄)-Alkylen oder (C₄-C₆)-Alkenylen, insbesondere CH₂CH₂, CH(CH₃)CH₂, C(CH₃)₂CH₂, CH(CH₃)CH(CH₃).

Besonders bevorzugt bedeutet die Gruppe
- -A-R³: (C₃-C₄)-Alkenyloxy-(C₂-C₄)-alkyl, (C₃-C₄)-Alkinyloxy-(C₂-C₄)-alkyl, Benzyloxy-(C₂-C₄)-alkyl, (C₃-C₄)-Alkenyloxycarbonyl-(C₁-C₄)-alkyl oder (C₃-C₄)-Alkinyloxycarbonyl-(C₁-C₄)-alkyl.

Bevorzugt sind erfindungsgemäße Verbindungen der Formel I, worin die Gruppe der Formel
- -A-R³: 2-(Allyloxy)-ethyl, 3-(Allyloxy)-n-propyl, 4-(Allyloxy)-n-butyl, 2-(Allyloxy)-1-methyl-ethyl, 2-(2-Methylprop-2-en-1-yl)-ethyl, 2-(Propargyloxy)-ethyl, 2-(Propargyloxy)-1-methyl-ethyl, 3-Propargyloxy-propyl, 4-Propargyloxybutyl, 2-Benzyloxy-ethyl, Allyloxycarbonylmethyl, 1-(Allyloxycarbonyl)-1-ethyl, 1-(Allyloxycarbonyl)-1,1-dimethylmethyl,
Propargyloxycarbonylmethyl, 1-(Propargyloxycarbonyl)-1-ethyl, 3-oder (2,2-Dimethyl-1,3-dioxolan-4-yl)-methyl bedeutet.

Gegenstand der Erfindung ist auch ein Verfahren zur Herstellung der erfindungsgemäßen Verbindungen der Formel I, dadurch gekennzeichnet, daß man
a) 5-Chlor-8-hydroxychinolin mit einem Alkancarbonsäurederivat der Formel II,

   Y-CR¹R²-CO-X-A-R³ II

   worin
   - Y: eine Abgangsgruppe, wie z.B. Chlor, Brom, Methansulfonyl oder Toluolsulfonyl bedeutet und
   - R¹, R², R³, X und A: wie bei der genannten Formel 1 definiert sind, oder
b) 5-Chlorchinolin-8-oxy-alkancarbonsäuren der Formel I, worin -X-A-R³ durch Hydroxy ersetzt ist, mit Alkoholen, Mercaptanen oder Aminen der Formel

   H - X - A - R³

   wobei X, A und R³ wie bei Formel I definiert sind,
umsetzt.

Die in Variante b) eingesetzten 5-Chlorchinolin-8-oxy-alkancarbonsäuren erhält man beispielsweise aus dem Ethylester, der nach Variante a) hergestellt werden kann, durch alkalische Hydrolyse.

Die Umsetzung der Verbindung II mit 5-Chlor-8-hydroxychinolin nach Variante a) wird vorzugsweise in dipolar aprotischen Lösungsmitteln, wie Dimethylsulfoxid oder N,N-Dimethylformamid, bei erhöhter Temperatur, insbesondere zwischen 80 und 120°C, in Gegenwart einer Base, insbesondere Alkalicarbonaten wie z.B. Kaliumcarbonat, durchgeführt.

Die Umsetzung nach Variante b) erfolgt vorzugsweise in dipolar aprotischen Lösungsmitteln insbesondere Ethern, wie z.B. Tetrahydrofuran oder 1,4-Dioxan, oder Halogenkohlenwasserstoffen, wie z.B. Chloroform oder Tetrachlorkohlenstoff, in Gegenwart eines die Carboxylgruppe in ein aktiviertes Derivat überführendes Reagenz, wie z.B. Thionylchlorid, N,N'-Carbonyldiimidazol oder Dicyclohexylcarbodiimid, bei Temperaturen von Raumtemperatur bis zum Siedepunkt des Reaktionsgemisches, insbesondere bei Rückflußtemperatur.

5-Chlor-8-hydroxychinolin ist kommerziell erhältlich. Die Bromalkancarbonsäurederivate der Formel II sind nach in der Literatur bekannten Verfahren aus Bromalkancarbonsäurechloriden und Verbindungen der Formel H-X-A-R³, wobei X, A und R³ wie in Formel I definiert sind, herstellbar. Alkohole, Mercaptane oder Amine der Formel H-X-A-R³ sind, sofern sie nicht ebenfalls kommerziell erhältlich sind, nach literaturbekannten Verfahren zugänglich; siehe z.B. Helv. Chim Acta 67, Seite 1470 ff. (1984); J. Am. Chem. Soc. 71, Seiten 1152 ff. (1949); J. Am. Chem. Soc. 60, Seiten 1472 ff. (1938); US-A-3 123 639; EP-A-52 798.

Verbindungen der Formel I reduzieren oder unterbinden phytotoxische Nebenwirkungen von Herbiziden, die beim Einsatz der Herbizide in Nutzpflanzenkulturen auftreten können, und können deshalb in üblicher Weise als Antidote oder Safener bezeichnet werden.

Die erfindungsgemäßen Verbindungen der Formel I können zusammen mit herbiziden Wirkstoffen oder in beliebiger Reihenfolge ausgebracht werden und sind dann in der Lage, schädliche Nebenwirkungen dieser Herbizide bei Kulturpflanzen zu reduzieren oder völlig aufzuheben, ohne die Wirksamkeit dieser Herbizide gegen Schadpflanzen zu beeinträchtigen.

Hierdurch kann das Einsatzgebiet herkömmlicher Pflanzenschutzmittel ganz erheblich erweitert werden. Herbizide, deren phytotoxische Nebenwirkungen auf Kulturpflanzen mittels Verbindungen der Formel I herabgesetzt werden können, sind z.B. Carbamate, Thiocarbamate, Halogenacetanilide, substituierte Phenoxy-, Naphthoxy- und Phenoxy-phenoxycarbonsäurederivate sowie Heteroaryloxy-phenoxyalkancarbonsäurederivate, wie Chinolyloxy-, Chinoxalyloxy-, Pyridyloxy-, Benzoxalyloxy- und Benzthiazolyloxy-phenoxyalkancarbonsäureester, Cyclohexandionabkömmlinge, Imidazolinone sowie Sulfonylharnstoffe. Bevorzugt sind dabei Phenoxyphenoxy- und Heteroaryloxy-phenoxycarbonsäureester und - salze, Sulfonylharnstoffe und Imidazolinone.

Geeignete Herbizide, die mit den erfindungsgemäßen Safenern kombiniert werden können sind beispielsweise:
A) Herbizide vom Typ der Phenoxyphenoxy- und Heteroarylphenoxycarbonsäure-(C₁-C₄)alkyl-, (C₂-C₄)alkenyl- und (C₃-C₄)alkinylester wie
   A1) Phenoxy-phenoxy- und Benzyloxy-phenoxy-carbonsäure-derivate, z.B.
      2-(4-(2,4-Dichlorphenoxy)-phenoxy)-propionsãuremethylester (Diclofop-methyl),
      2-(4-(4-Brom-2-chlorphenoxy)-phenoxy)-propionsäuremethylester (s. DE-A-2601548),
      2-(4-(4-Brom-2-fluorphenoxy)-phenoxy)-propionsäuremethylester (s. US-A-4808750),
      2-(4-(2-Chlor-4-trifluormethylphenoxy)-phenoxy)-propionsäuremethylester (s. DE-A-2433067),
      2-(4-(2-Fluor-4-trifluormethylphenoxy)-phenoxy)-propionsäuremethylester (s. US-A-4808750),
      2-(4-(2,4-Dichlorbenzyl)-phenoxy)propionsäuremethylester (s. DE-A-2417487), 4-(4-(4-Trifluormethylphenoxy)-phenoxy)-pent-2-en-säureethylester,
      2-(4-(4-Trifluormethylphenoxy)-phenoxy)-propionsäuremethylester (s. DE-A-2433067),
   A2) "Einkernige" Heteroaryloxy-phenoxy-alkancarbonsäurederivate, z.B.
      2-(4-(3,5-Dichlorpyridyl-2-oxy)-phenoxy)-propionsäureethylester (s. EP-A-2925),
      2-(4-(3,5-Dichlorpyridyl-2-oxy)-phenoxy)-propionsäurepropargylester (EP-A-3114),
      2-(4-(3-Chlor-5-trifluormethyl-2-pyridyloxy)-phenoxy-propionsäure-methylester (s. EP-A-3890),
      2-(4-(3-Chlor-5-trifluormethyl-2-pyridyloxy)-phenoxy)-propionsäure-ethylester (s. EP-A-3890),
      2-(4-(5-Chlor-3-fluor-2-pyridyloxy)-phenoxy)-propionsäurepropargylester (EP-A-191736),
      2-(4-(5-Trifluormethyl-2-pyridyloxy)-phenoxy)-propionsäurebutylester (Fluazifopbutyl),
   A3) "Zweikernige" Heteroaryloxy-phenoxy-alkancarbonsäurederivate, z.B.
      2-(4-(6-Chlor-2-chinoxalyloxy)-phenoxy)-propionsäuremethylester und -ethylester (Quizalofop-methyl und -ethyl),
      2-(4-(6-Fluor-2-chinoxalyloxy)-phenoxy)-propionsäuremethylester (s. J. Pest. Sci. Vol. 10, 61 (1985)),
      2-(4-(6-Chlor-2-chinoxalyloxy)-phenoxy)-propionsäure, -methylester, -tetrahydrofurfuryl-, und -2-isopropylidenaminooxyethylester (Propaquizafop u. verschiedenste Ester),
      2-(4-(6-Chlorbenzoxazol-2-yl-oxy)-phenoxy)-propionsäureethylester (Fenoxapropethyl),
      2-(4-(6-Chlorbenzthiazol-2-yloxy)phenoxypropionsäureethylester (s. DE-A-2640730).
B) Herbizide aus der Sulfonylharnstoff-Reihe, wie z.B. Pyrimidin- oder Triazinylaminocarbonyl-[benzol-, pyridin-, pyrazol-, thiophen- und (alkylsulfonyl)alkylamino-]-sulfamide. Bevorzugt als Substituenten am Pyrimidinring oder Triazinring sind Alkoxy, Alkyl, Haloalkoxy, Haloalkyl, Halogen oder Dimethylamino, wobei alle Substituenten unabhängig voneinander kombinierbar sind. Bevorzugte Substituenten im Benzol-, Pyridin-, Pyrazol-, Thiophen- oder (Alkylsulfonyl)alkylamino-Teil sind Alkyl, Alkoxy, Halogen, Nitro, Alkoxycarbonyl, Aminocarbonyl, Alkylaminocarbonyl, Dialkylaminocarbonyl, Alkoxyaminocarbonyl, Alkyl, Alkoxyaminocarbonyl, Haloalkoxy, Haloalkyl, Alkylcarbonyl, Alkoxyalkyl, (Alkansulfonyl)alkylamino. Geeignete Sulfonylharnstoffe sind beispielsweise
   B1) Phenyl- und Benzylsulfonylharnstoffe und verwandte Verbindungen, z.B.
      1-(2-Chlorphenylsulfonyl)-3-(4-methoxy-6-methyl-1,3,5-triazin-2-yl)harnstoff (Chlorsulfuron),
      1-(2-Ethoxycarbonylphenylsulfonyl)-3-(4-chlor-6-methoxypyrimidin-2-yl)harnstoff (Chlorimuron-ethyl),
      1-(2-Methoxyphenylsulfonyl)-3-(4-methoxy-6-methyl-1,3,5-triazin-2-yl)harnstoff (Metsulfuron-methyl),
      1-(2-Chlorethoxy-phenylsulfonyl)-3-(4-methoxy-6-methyl-1,3,5-triazin-2-yl)harnstoff (Triasulfuron),
      1-(2-Methoxycarbonyl-phenylsulfonyl)-3-(4,6-dimethyl-pyrimidin-2-yl)harnstoff (Sulfometuron-methyl,
      1-(2-Methoxycarbonylphenylsulfonyl)-3-(4-methoxy-6-methyl-1,3,5-triazin-2-yl)-3-methylharnstoff (Tribenuron-methyl)
      1-(2-Methoxycarbonylbenzylsulfonyl)-3-(4,6-dimethoxy-pyrimidin-2-yl)harnstoff (Bensulfuron-methyl)
      1-(2-Methoxycarbonylphenylsulfonyl)-3-(4,6-bis-(difluormethoxy)pyrimidin-2-yl)harnstoff (Primisulfuron-methyl),
      3-(4-Ethyl-6-methoxy-1,3,5-triazin-2-yl)-1-(2,3-dihydro-1,1-dioxo-2-methylbenzo[b]thiophen-7-sulfonyl)-harnstoff (s. EP-A-79683),
      3-(4-Ethoxy-6-ethyl-1,3,5-triazin-2-yl)-1-(2,3-dihydro-1,1-dioxo-2-methylbenzo[b]thiophen-7-sulfonyl)-harnstoff (s. EP-A-79683),
   B2) Thienylsulfonylharnstoffe, z.B.
      1-(2-Methoxycarbonylthiophen-3-yl)-3-(4-methoxy-6-methyl-1,3,5-triazin-2-yl)harnstoff (Thifensulfuron-methyl),
   B3) Pyrazolylsulfonylharnstoffe, z.B.
      1-(4-Ethoxycarbonyl-1-methylpyrazol-5-yl-sulfonyl)-3-(4,6-dimethoxypyrimidin-2-yl)harnstoff (Pyrazosulfuron-methyl),
      Methyl-3-chlor-5-(4,6-dimethoxypyrimidin-2-ylcarbamoylsulfamoyl)-1-methyl-pyrazol-4-carboxylat (s. EP 282613),
   B4) Sulfondiamid-Derivate, z.B.
      3-(4,6-Dimethoxypyrimidin-2-yl)-1-(N-methyl-N-methylsulfonylaminosulfonyl)harnstoff (Amidosulfuron) und Strukturanaloge (s. EP-A-0131258 und Z. Pfl. Krankh. Pfl. Schutz, Sonderheft XII, 489-497 (1990)),
   B5) Pyridylsulfonylharnstoffe, z.B.
      1-(3-N,N-Dimethylaminocarbonylpyridin-2-yl-sulfonyl)-3-(4,6-dimethoxypyrimidin-2-yl)harnstoff (Nicosulfuron),
      1-(3-Ethylsulfonylpyridin-2-yl-sulfonyl)-3-(4,6-dimethoxy-pyrimidin-2-yl)harnstoff (DPX-E 9636, s. Brighton Crop Prot. Conf. - Weeds - 1989, S. 23 ff.),
      Pyridylsulfonylharnstoffe, wie sie in WO 91/10660 und der deutschen Patentanmeldung P 4030577.5 beschrieben sind, vorzugsweise solche der Formel III oder deren Salze, 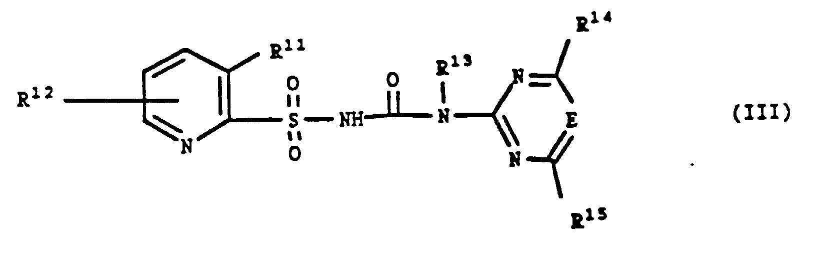
      worin
      - E: CH oder N vorzugsweise CH,
      - R¹¹: lod oder NR¹⁶R¹⁷,
      - R¹²: H, Halogen, Cyano, C₁-C₃-Alkyl, C₁-C₃-Alkoxy, C₁-C₃-Haloalkyl, C₁-C₃-Haloalkoxy, C₁-C₃-Alkylthio, (C₁-C₃-Alkoxy)-C₁-C₃-alkyl, (C₁-C₃-Alkoxy)-carbonyl, Mono- oder Di-(C₁-C₃-alkyl)-amino, C₁-C₃-Alkyl-sulfinyl oder -sulfonyl, SO₂-NR^{a}R^{b} oder CO-NR^{a}R^{b}, insbesondere H
      - R^{a},R^{b}: unabhängig voneinander H, C₁-C₃-Alkyl, C₁-C₃-Alkenyl, C₁-C₃-Alkinyl oder zusammen -(CH₂)₄-, -(CH₂)₅-oder-(CH₂)₂-O-(CH₂)₂-,
      - R¹³: H oder CH₃,
      - R¹⁴: Halogen, C₁-C₂-Alkyl, C₁-C₂-Alkoxy, C₁-C₂-Haloalkyl, vorzugsweise CF₃, C₁-C₂-Haloalkoxy, vorzugsweise OCHF₂ oder OCH₂CF₃,
      - R¹⁵: C₁-C₂-Alkyl, C₁-C₂-Haloalkoxy, vorzugsweise OCHF₂, oder C₁-C₂-Alkoxy, und
      - R¹⁶: C₁-C₄-Alkyl und R¹⁷ C₁-C₄-Alkylsulfonyl oder R¹⁶ und R¹⁷ gemeinsam eine Kette der Formel -(CH₂)₃SO₂- oder -(CH₂)₄SO₂ bedeuten, z.B. 3-(4,6-Dimethoxypyrimidin-2-yl)-1-(3-N-methylsulfonyl-N-methylaminopyridin-2-yl)sulfonylharnstoff,
   B6) Alkoxyphenoxysulfonylharnstoffe, wie sie in EP-A-0342569 beschrieben sind, vorzugsweise solche der Formel IV oder deren Salze, 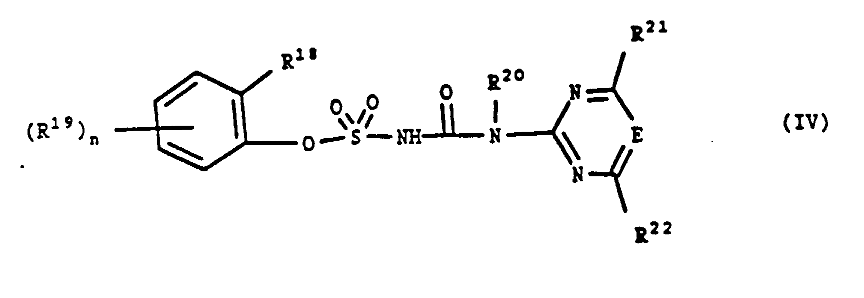 worin
      - E: CH oder N, vorzugsweise CH,
      - R¹⁸: Ethoxy, Propoxy oder Isopropoxy,
      - R¹⁹: Wasserstoff, Halogen, NO₂, CF₃, CN, C₁-C₄-Alkyl, C₁-C₄-Alkoxy, C₁-C₄-Alkylthio oder (C₁-C₃-Alkoxy)-carbonyl, vorzugsweise in 6-Position am Phenylring,
      - n: 1, 2 oder 3, vorzugsweise 1,
      - R²⁰: Wasserstoff, C₁-C₄-Alkyl oder C₃-C₄-Alkenyl,
      - R²¹,R²²: unabhängig voneinander Halogen, C₁-C₂-Alkyl, C₁-C₂-Alkoxy, C₁-C₂-Haloalkyl, C₁-C₂-Haloalkoxy oder (C₁-C₂-Alkoxy)-C₁-C₂-alkyl, vorzugsweise OCH₃ oder CH₃, bedeuten, z.B. 3-(4,6-Dimethoxypyrimidin-2-yl)-1-(2-ethoxyphenoxy)sulfonylharnstoff,
      und andere verwandte Sulfonylharnstoffderivate und Mischungen daraus;
C) Chloracetanilid-Herbizide wie
   N-Methoxymethyl-2,6-diethyl-chloracetanilid (Alachlor),
   N-(3'-Methoxyprop-2'-yl)-2-methyl-6-ethyl-chloracetanilid (Metolachlor),
   N-(3-Methyl-1,2,4-oxdiazol-5-yl-methyl)-chloressigsäure-2,6-dimethylanilid,
   N-(2,6-Dimethylphenyl)-N-(1-pyrazolylmethyl)-chloressigsäureamid (Metazachlor),
D) Thiocarbamate wie
   S-Ethyl-N,N-dipropylthiocarbamat (EPTC) oder
   S-Ethyl-N,N-diisobutylthiocarbamat (Butylate);
E) Cyclohexandion-Derivate wie
   Methyl-3-(1-allyloxyimino)butyl)-4-hydroxy-6,6-dimethyl-2-oxocyclohex-3-encarboxylat (Alloxydim);
   2-(N-Ethoxybutyrimidoyl)-5-(2-ethylthiopropyl)-3-hydroxy-2-cyclohexen-1-on (Sethoxydim),
   2-(N-Ethoxybutyrimidoyl)-5-(2-phenylthiopropyl)-3-hydroxy-2-cyclohexen-1-on (Cloproxydim),
   2-(1-(3-Chlorallyloxy)iminobutyl)-5-(2-ethylthio)propyl)-3-hydroxy-2-cyclohexen-1-on,
   2-(1-(3-Chlorallyloxy)iminopropyl)-5-2-ethylthio)propyl)-3-hydroxy-cyclohex-2-enon (Clethodim),
   2-(1-Allyloxyiminobutyl)-4-methoxycarbonyl-5,5-dimethyl-3-oxocyclohexenol,
   2-(1-(Ethoxyimino)-butyl)-3-hydroxy-5-(thian-3-yl)-cyclohex-2-enon (Cycloxydim) oder
   2-(1-Ethoxyiminopropyl)-5-(2,4,6-trimethylphenyl)-3-hydroxy-2-cyclohexen-1-on (Tralkoxydim);
F) 2-Carboxyphenyl- oder 2-Carboxyheteroaryl-imidazolinone, deren Salze und Ester (z.B. Alkylester), z.B. die Mischung von 2-(4-Isopropyl-4-methyl-5-oxo-2-imidazolin-2-yl)-5-methylbenzoesäuremethylester und 2-(4-Isopropyl-4-methyl-5-oxo-2-imidazolin-2-yl)-4-methylbenzoesäuremethylester (Imazamethabenz), 5-Ethyl-2-(4-isopropyl-4-methyl-5-oxo-2-imidazolin-2-yl)-pyridin-3-carbonsäure (Imazethapyr), deren Ester und Salze (z. B. NH₄-Salz), 2-(4-Isopropyl-4-methyl-5-oxo-2-imidazolin-2-yl)-chinolin-3-carbonsäure (Imazaquin), deren Ester und Salze (z.B. NH₄-salz) und rac-2-[4,5-dihydro-4-methyl-4-(1-methylethyl)-5-oxo-1H-imidazol-2-yl]-5-methyl-3-pyridin-carbonsäure (Imazethamethapyr), deren Ester und Salze.

Die obengenannten Herbizide der Gruppe A bis F sind dem Fachmann bekannt und in der Regel in "The Pesticide Manual", British Crop Protection Council, 9. Auflage 1991 oder 8. Auflage 1987 oder in "Agricultural Chemicals Book II, Herbicides", by W.T. Thompson, Thompson Publications, Fresno CA, USA 1990 oder in "Farm Chemicals Handbook '90", Meister Publishing Company, Willoughby OH, USA 1990 beschrieben. Imazethamethapyr ist aus Weed Techn. 1991, Vol. 5, 430-438 bekannt.

Die herbiziden Wirkstoffe und die erwähnten Safener können zusammen (als fertige Formulierung oder im Tank-mix-Verfahren) oder in beliebiger Reihenfolge nacheinander ausgebracht werden. Das Gewichtsverhältnis Safener:Herbizid kann innerhalb weiter Grenzen variieren und ist vorzugsweise im Bereich von 1:10 bis 10:1, insbesondere von 1:10 bis 5:1. Die jeweils optimalen Mengen an Herbizid und Safener sind vom Typ des verwendeten Herbizids oder vom verwendeten Safener sowie von der Art des zu behandelnden Pflanzenbestandes abhängig und lassen sich von Fall zu Fall durch entsprechende Vorversuche ermitteln.

Haupteinsatzgebiete für die Anwendung der Safener sind vor allem Getreidekulturen (Weizen, Roggen, Gerste, Hafer), Reis, Mais, Sorghum, aber auch Baumwolle und Sojabohne, vorzugsweise Getreide und Mais.

Ein besonderer Vorteil der erfindungsgemäßen Safener der Formel I ist bei deren Kombination mit Herbiziden aus der Gruppe der Sulfonylharnstoffen und/oder Imidazolinone festzustellen. Herbizide der genannten Strukturklassen hemmen primär das Schlüsselenzym Acetolactatsynthase (ALS) in den Pflanzen und sind bezüglich des Wirkungsmechanismus daher zumindest partiell verwandt. Einige Herbizide dieser Strukturklassen können speziell in Getreidekulturen und/oder Mais nicht oder nicht genügend selektiv eingesetzt werden. Durch die Kombination mit den erfindungsgemäßen Safenern sind auch bei diesen Herbiziden in Getreide oder Mais hervorragende Selektivitäten zu erreichen.

Die Safener der Formel I je nach ihren Eigenschaften zur Vorbehandlung des Saatgutes der Kulturpflanze (Beizung der Samen) verwendet werden oder vor der Saat in die Saatfurchen eingebracht oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein. Bevorzugt ist die gemeinsame Anwendung mit dem Herbizid. Hierzu können Tankmischungen oder Fertigformulierungen eingesetzt werden.

Die benötigten Aufwandmengen der Safener können je nach Indikation und verwendetem Herbizid innerhalb weiter Grenzen schwanken und sind in der Regel im Bereich von 0,001 bis 5 kg, vorzugsweise 0,005 bis 0,5 kg Wirkstoff je Hektar.

Gegenstand der vorliegenden Erfindung ist deshalb auch ein Verfahren zum Schutz von Kulturpflanzen vor phytotoxischen Nebenwirkungen von Herbiziden, das dadurch gekennzeichnet ist, daß eine wirksame Menge einer Verbindung der Formel I vor, nach oder gleichzeitig mit dem Herbizid auf die Pflanzen, Pflanzensamen oder die Anbaufläche appliziert wird.

Gegenstand der Erfindung sind auch pflanzenschützende Mittel, die einen Wirkstoff der Formel I und übliche Formulierungshilfsmittel enthalten, sowie herbizide Mittel, die einen Wirkstoff der Formel I und ein Herbizid sowie im Bereich des Pflanzenschutzes übliche Formulierungshilfsmittel enthalten.

Die Verbindungen der Formel I und deren Kombinationen mit einem oder mehreren der genannten Herbizide können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), emulgierbare Konzentrate (EC), wasserlösliche Pulver (SP), wasserlösliche Konzentrate (SL), konzentrierte Emulsionen (EW) wie Öl-in-Wasser und Wasser-in- Öl-Emulsionen, versprühbare Lösungen oder Emulsionen, Kapselsuspensionen (CS), Dispersionen auf Öl- oder Wasserbasis (SC), Suspoemulsionen, Suspensionskonzentrate, Stäubemittel (DP), ölmischbare Lösungen (OL), Beizmittel, Granulate (GR) in Form von Mikro-, Sprüh-, Aufzugs- und Adsorptionsgranulaten, Granulate für die Boden- bzw. Streuapplikation, wasserlösliche Granulate (SG), wasserdispergierbare Granulate (WG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie" Band 7, C. Hauser Verlag München, 4. Aufl. 1986; Wade van Valkenburg, "Pesticide Formulations", Marcel Dekker N.Y., 1973; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen "Intruduction to Clay Colloid Chemistry", 2nd Ed., J. Wiley & Sons, N.Y. Marsden "Solvents Guide", 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler "Chemische Technolgie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole und Fettamine, Fettalkoholpolyglykolethersulfate, Alkansulfonate oder Alkylarylsulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid Kondensationsprodukte (z.B. Blockpolymere), Alkylpolyether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit und Pyrophillit, oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Die agrochemischen Zubereitungen enthalten in der Regel 0,1 bis 99 Gewichtsprozent, insbesondere 0,1 bis 95 Gew.-%, Wirkstoffe der Formel I (Antidot) oder des Antidot/Herbizid-Wirkstoffgemischs und 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

In Spritzpulvern beträgt die Wirkstoffkonzentration z.B. etwa 10 bis 90 Gew.%, der Rest zu 100 Gew.-% besteht aus üblichen Formulierungsbestandteilen. Bei emulgierbaren Konzentraten beträgt die Wirkstoffkonzentration etwa 1 bis 80 Gew.-% Wirkstoffe. Staubförmige Formulierungen enthalten etwa 1 bis 20 Gew.-% an Wirkstoffen, versprühbare Lösungen etwa 0,2 bis 20 Gew.-% Wirkstoffe. Bei Granulaten wie wasserdispergierbaren Granulaten hängt der Wirkstoffgehalt zum Teil davon ab, ob die wirksame Verbindung flüssig oder fest vorliegt. In der Regel liegt der Gehalt bei den in Wasser dispergierbaren Granulaten zwischen 10 und 90 Gew.-%.
Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Granulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids u.a. variiert die erforderliche Aufwandmenge der "Antidots".

Folgende Beispiele dienen zur Erläuterung der Erfindung:

### A. Formulierungsbeispiele

a) Ein Stäubmittel wird erhalten, indem man 10 Gew.-Teile einer Verbindung der Formel I oder eines Wirkstoffgemischs aus einem Herbizid und eine Verbindung der Formel I und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel I oder eines Wirkstoffgemischs aus einem Herbizid und einem Safener der Formel I, 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel I oder eines Wirkstoffgemischs aus einem Herbizid und einem Safener der Formel I, 6 Gew.-Teilen Alkylphenolpolyglykolether (^{R}Triton X 207), 3 Gew.-Teilen Isotridecanolpolyglykolether (8 EO) und 71 Gew.- Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel I oder eines Wirkstoffgemischs aus einem Herbizid und einem Safener der Formel 1, 75 Gew.Teilen Cyclohexanon als Lösemittel und 10 Gew.-Teilen oxethyliertes Nonylphenol als Emulgator.
e) Ein in Wasser dispergierbares Granulat wird erhalten, indem man

| | |
|---|---|
| 75 Gew.-Teile | einer Verbindung der Formel I oder eines Wirkstoffgemischs aus einem Herbizid und einem Safener der Formel I, |
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man

| | |
|---|---|
| 25 Gew.-Teile | einer Verbindung der Formel I oder eines Wirkstoffgemischs aus einem Herbizid und einem Safener der Formel I, |
| 5 " | 2,2'-dinaphthylmethan-6,6'-disulfonsaures Natrium, |
| 2 " | oleoylmethyltaurinsaures Natrium, |
| 1 " | Polyvinylalkohol, |
| 17 " | Calciumcarbonat und |
| 50 " | Wasser |

auf einer Kolloidmühle homogensiert und vorzerkleinert, anschließend auf einer Perlmühle mahlt und die so erhaltene Suspension in einem Sprühturm mittels einer Einstoffdüse zerstäubt und trocknet.

### B. Herstellungbeispiele

### 5-Chlorchinolin-8-oxyessigsäure-3-(allyloxy)propylester (Bsp. 19 in Tabelle 1)

3,78 g (0,021 mol) 5-Chlor-8-hydroxychinolin und 2,91 g (0,021 mol) Kaliumcarbonat werden in 100 ml Dimethylsulfoxid (DMSO) für 30 min auf 60°C erwärmt. Man läßt wieder auf Raumtemperatur abkühlen und tropft dann 5,0 g (0,021 mol) Bromessigsäure-3-(allyloxy)propylester hinzu und erwärmt die Lösung anschließend für 4 h auf 90°C. Das DMSO wird dann im Vakuum abdestilliert, der Rückstand in Essigsäureethylester aufgenommen und die Lösung mit Wasser und 5 proz. Natriumhydroxidlösung gewaschen. Die organische Phase wird über Magnesiumsulfat getrocknet, das Trockenmittel abfiltriert und das Lösungsmittel unter reduziertem Druck abgezogen. Nach Umkristallisieren des Rückstandes aus n-Heptan erhält man 5,4 g (76,3 % d. Th.) 5-Chlorchinolin-8-oxyessigsäure-3-(allyloxy)propylester vom Schmp. 69°C.

### 5-Chlorchinolin-8-oxyessigsäure-2-(propargyloxy)ethylester (Bsp. 18 in Tabelle 1)

5,0 g (0,021 mol) 5-Chlorchinolin-8-oxyessigsäure werden in 70 ml Thionylchlorid eine Stunde lang auf 70°C erwärmt. Anschließend wird das überschüssige Thionylchlorid im Vakuum abdestilliert und der Rückstand in 150 ml Tetrachlorkohlenstoff suspendiert. Zu dieser Suspension fügt man 2,10 g (0,021 mol) 2-Propargyloxyethanol hinzu, tropft dann 2,30 g (0,023 mol) Triethylamin hinzu und erhitzt 12 h zum Rückfluß. Anschließend wäscht man die Suspension mit je 70 ml 2 n HCl und 5 proz. Natronlauge, trocknet die org. Phase über Magnesiumsulfat und zieht das Lösungsmittel i. Vak. ab. Der Rückstand wird aus n-Heptan umkristallisiert. Man erhält so 1,1 g (16,3 % d. Th.) 5-Chlorchinolin-8-oxyessigsäure-2-(propargyloxy)ethylester vom Schmp. 53°C.

### 5-Chlorchinolin-8-oxyessigsäure-2-allyloxy-1-methylethylester (Bsp. 24 in Tabelle 1)

5,0 g (0,021 mol) 5-Chlorchinolin-8-oxyessigsäure und 2,44 g (0,021 mol) 2-Allyloxy-1-methylethanol werden in einem Gemisch aus 40 ml Dichlormethan und 40 ml Dimethylformamid suspendiert und auf 0°C abgekühlt. Bei dieser Temperatur werden 4,78 g (0,023 mol) Dicyclohexylcarbodiimid in 10 ml Dichlormethan gelöst hinzugetropft und dann 200 mg 3-(N,N-Dimethylamino)-pyridin hinzugegeben. Man rührt 15 h bei Raumtemperatur und saugt den ausgefallenen Niederschlag ab und wäscht ihn mit 50 ml Dichlormethan nach. Das Filtrat wird mit 100 ml 0,5 n HCl, mit 100 ml Kaliumhydrogencarbonatlösung und 3 mal mit je 50 ml Wasser gewaschen. Die organische Phase wird über Magnesiumsulfat getrocknet und das Lösungsmittel im Vakuum abgezogen. Der Rückstand wird aus n-Heptan umkristallisiert. Auf diese Weise erhält man 5,1 g (72,4 % d. Th.) 5-Chlorchinolin-8-oxyessigsäure-2-allyloxy-1-methylethylester vom Schmelzpunkt 59°C.

In den folgenden Tabellen 1a und 1b sind die obengenannten Herstellungsbeispiele mit weiteren Beispielen für Verbindungen der Formel I aufgeführt, die in analoger Weise hergestellt werden.

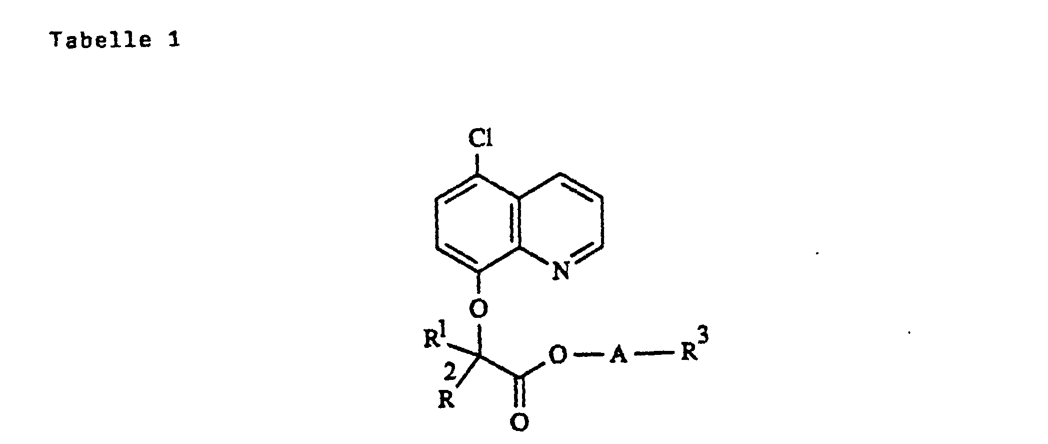

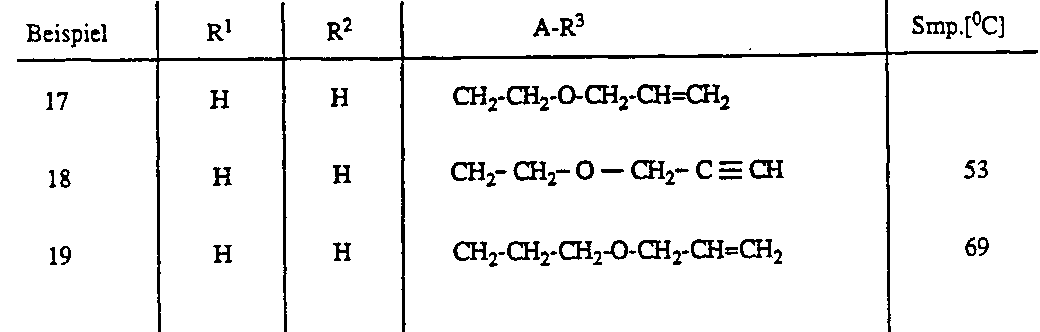

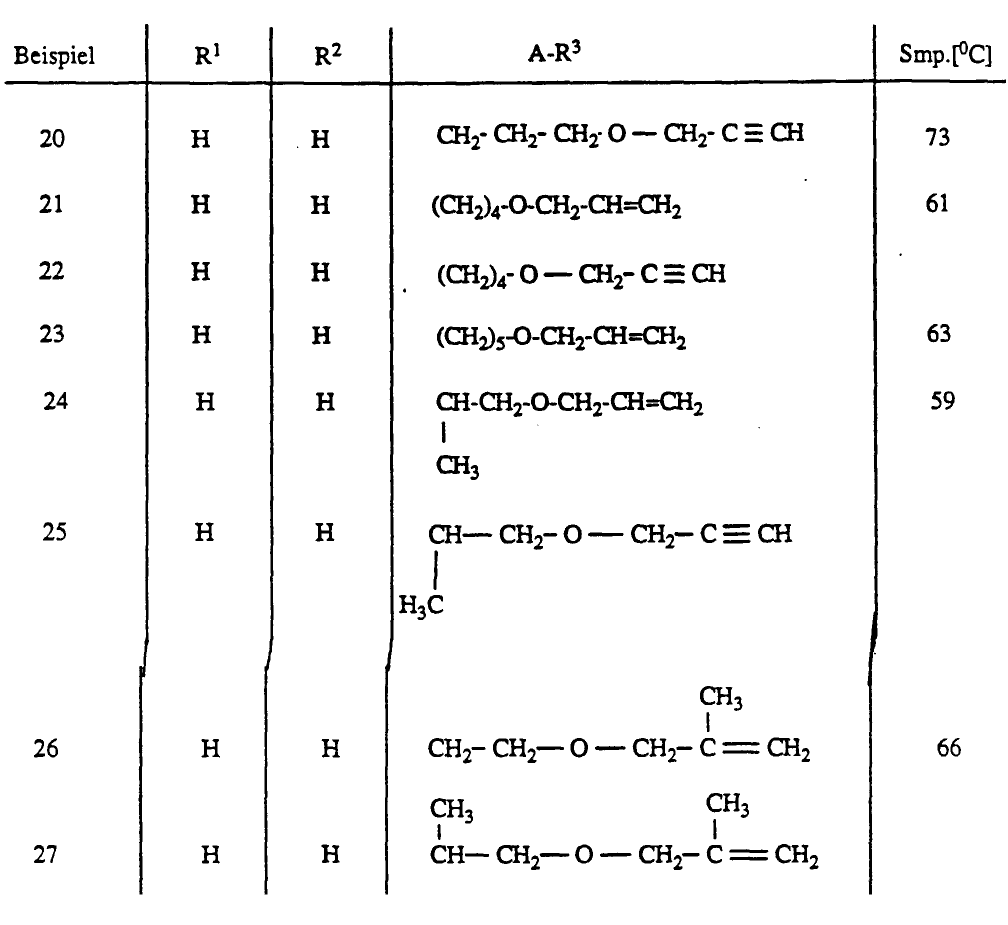

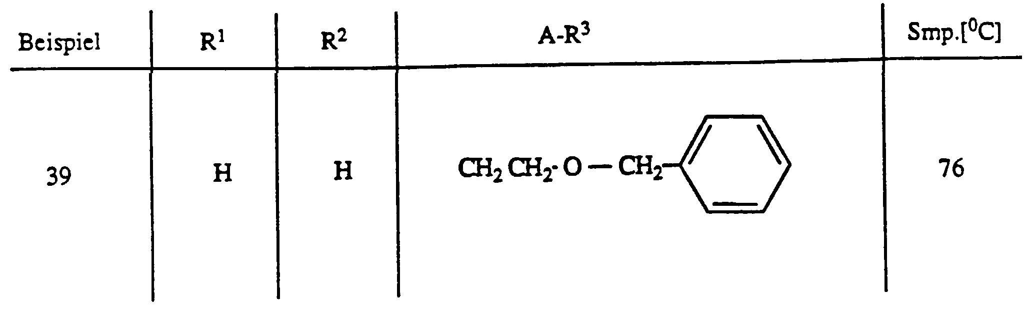

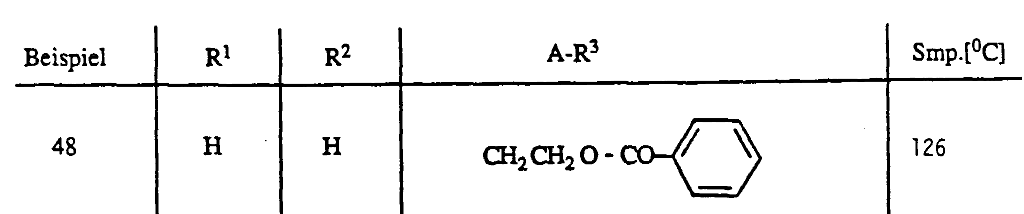

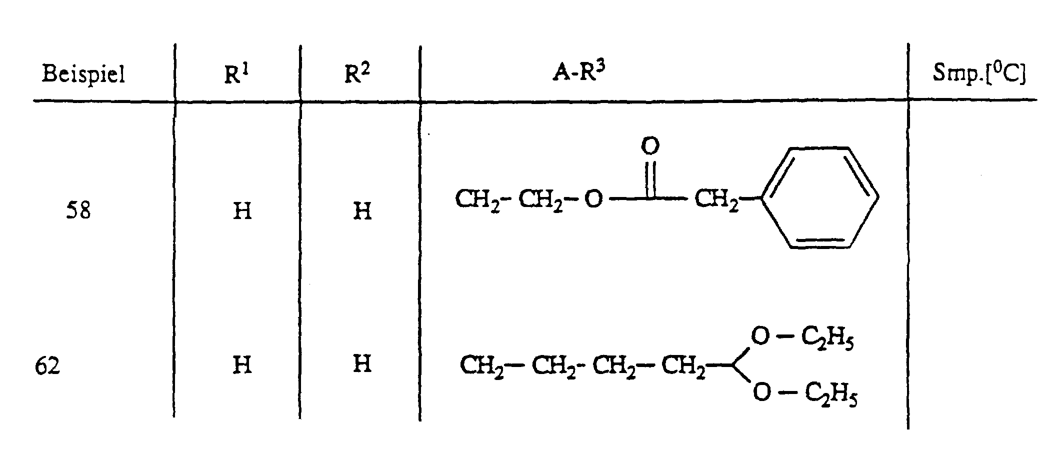

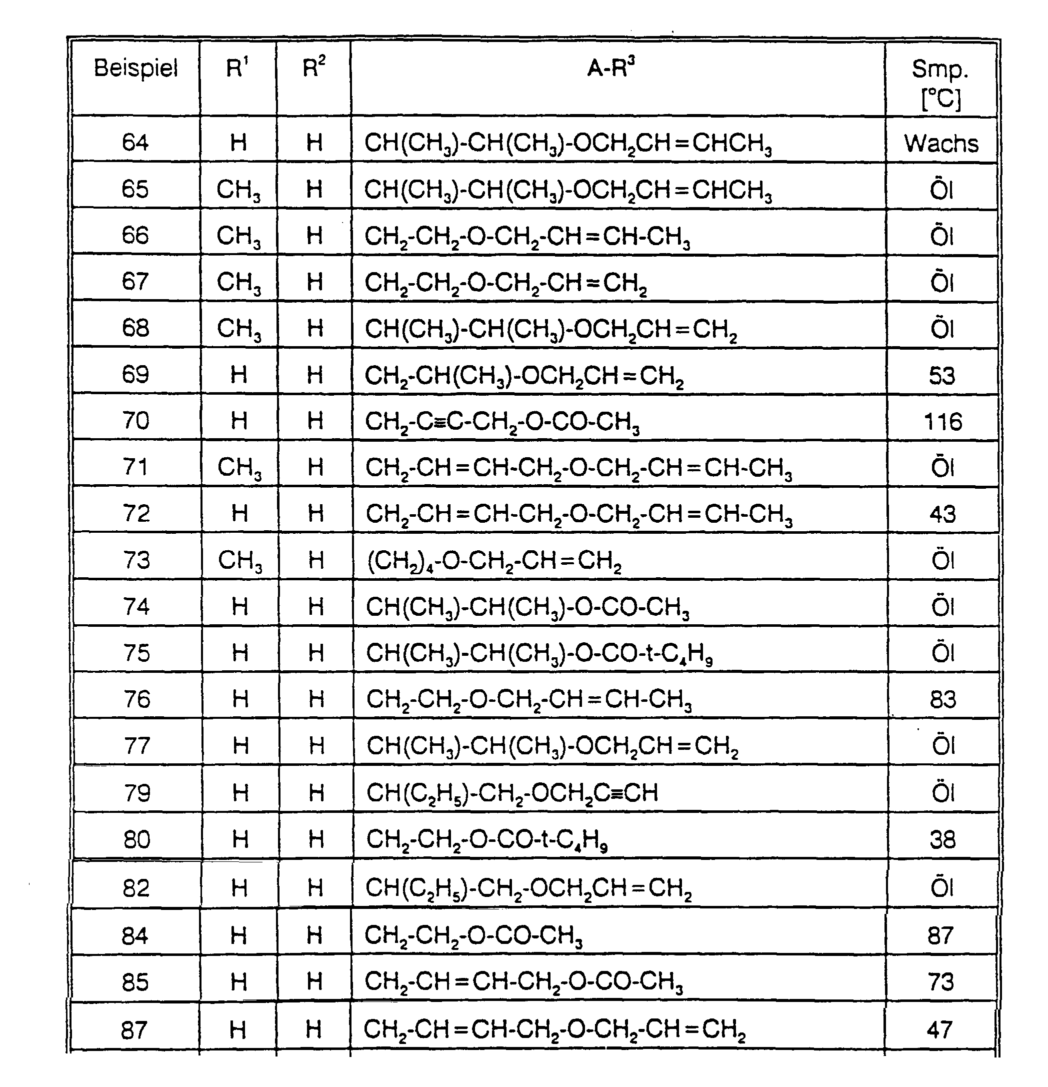

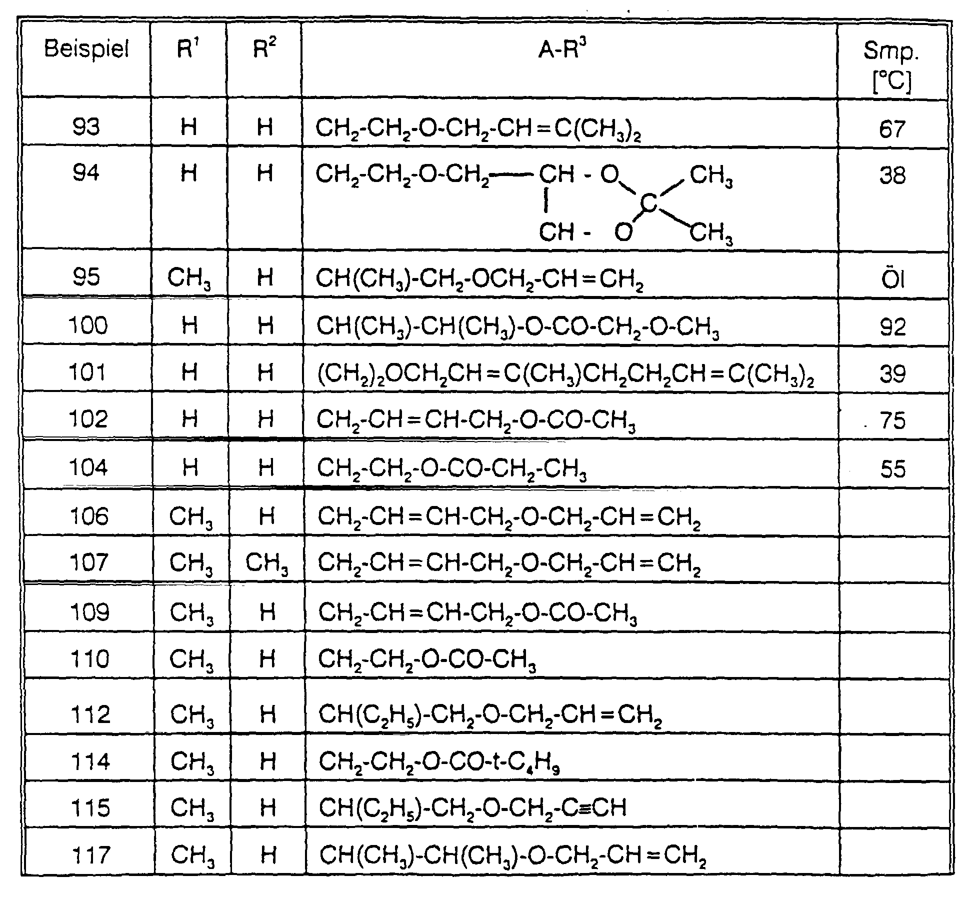

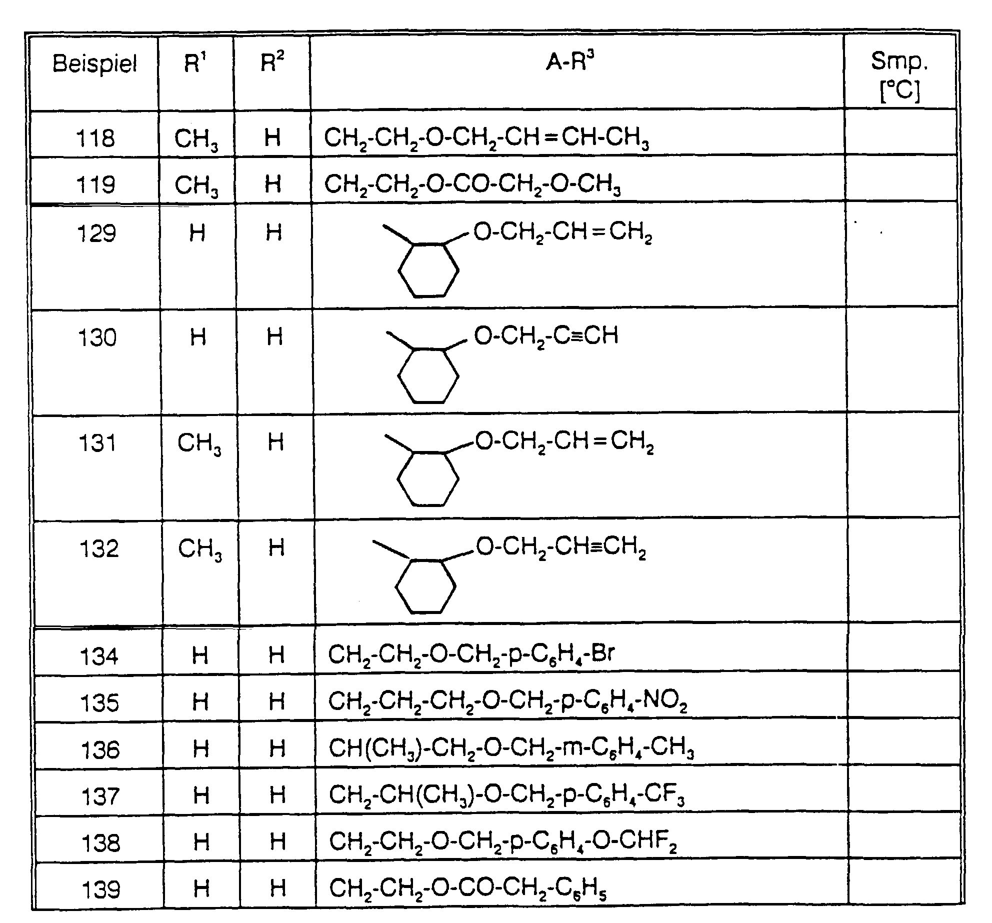

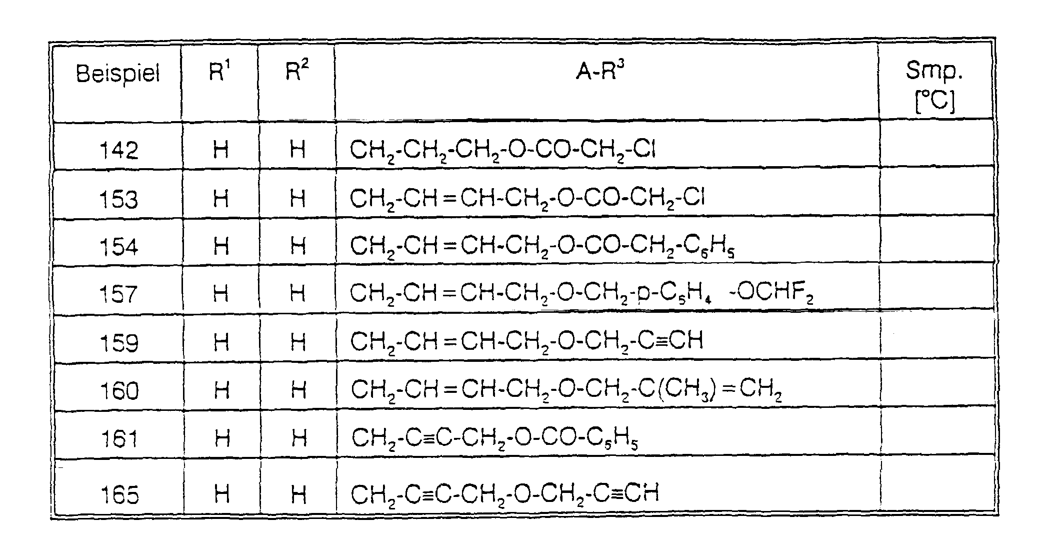

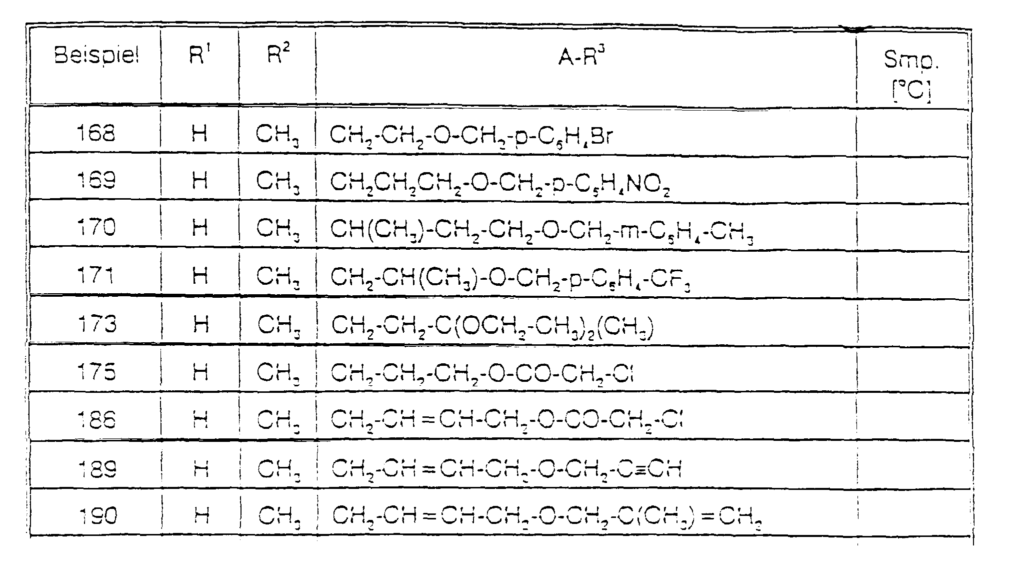

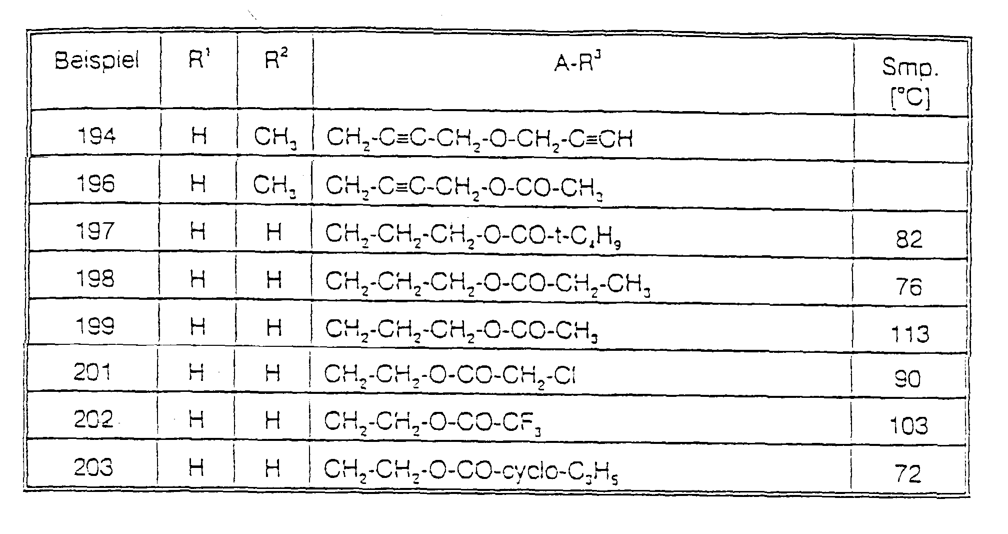

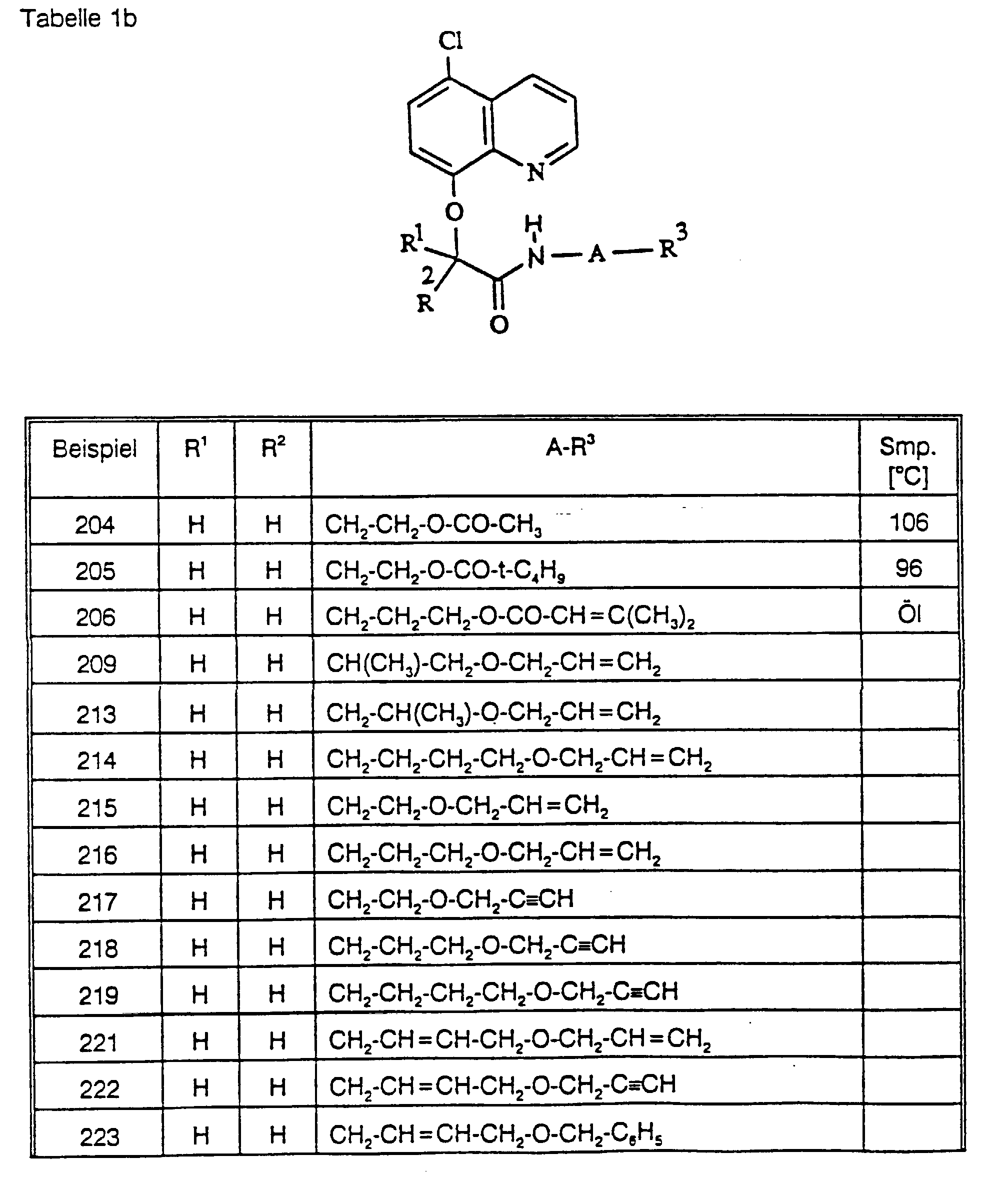

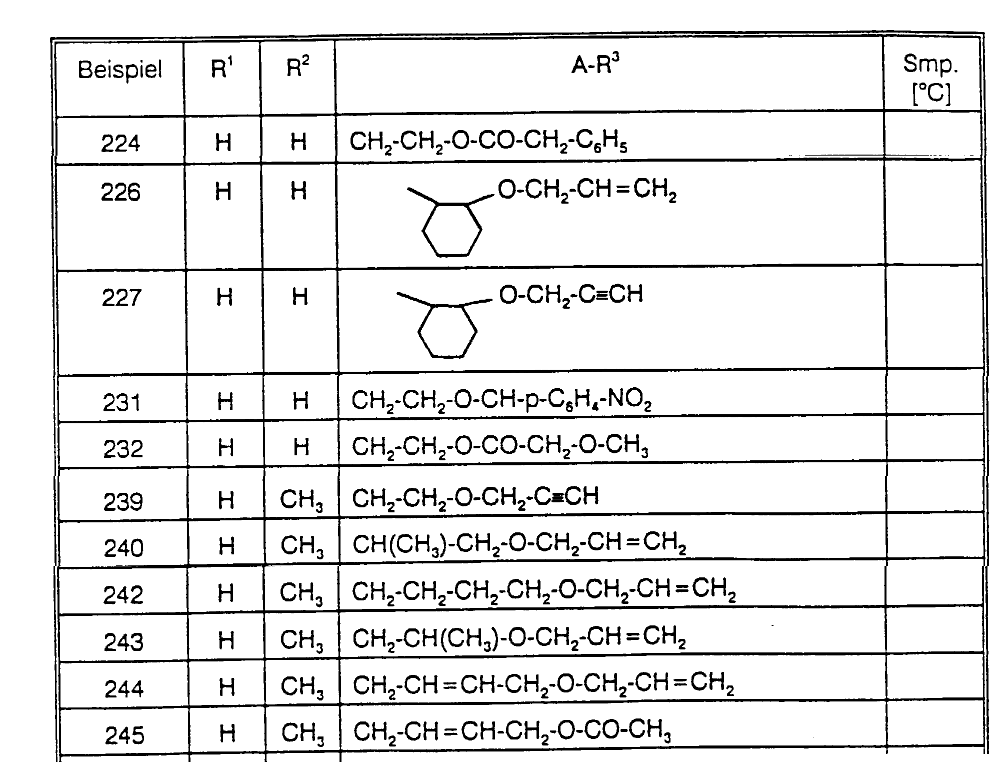

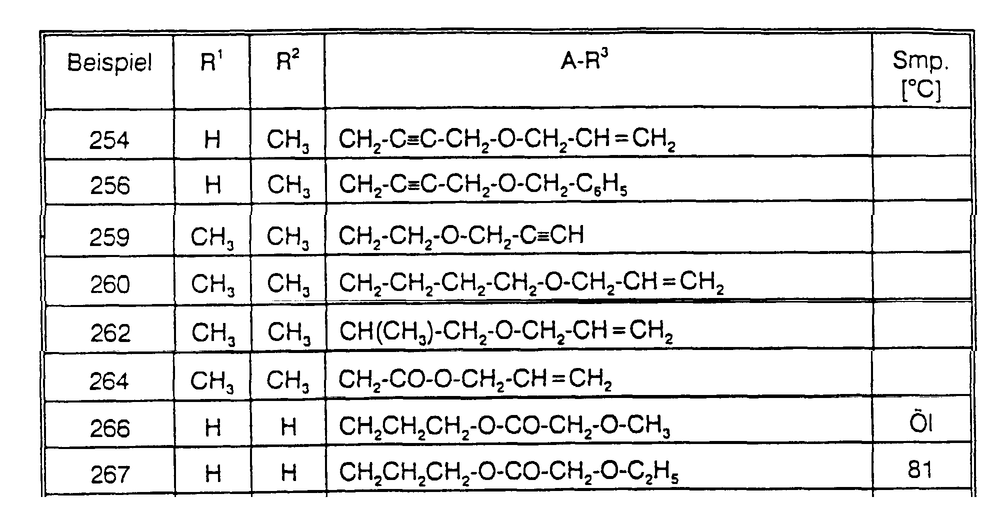

### C. Biologische Beispiele

### Beispiel 1

Weizen und Gerste wurden im Gewächshaus in Plastiktöpfen bis zum 34 Blattstadium herangezogen und dann nacheinander mit den erfindungsgemäßen Verbindungen und den getesteten Herbiziden im Nachauflaufverfahren behandelt. Die Herbizide und die Verbindungen der Formel I wurden dabei in Form wässriger Suspensionen bzw. Emulsionen mit einer Wasseraufwandmenge von umgerechnet 300 l/ha ausgebracht. 3-4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde. Die Bewertung erfolgte in Prozentwerten im Vergleich zu unbehandelten Kontrollen.

Die Ergebnisse aus Tabelle 2 veranschaulichen, daß die erfindungsgemäßen Verbindungen starke Herbizidschäden an Kulturpflanzen effektiv reduzieren können.

Selbst bei starken Überdosierungen des Herbizids werden bei den Kulturpflanzen auftretende schwere Schädigungen deutlich reduziert, geringere Schäden völlig aufgehoben.
Mischungen aus Herbiziden und erfindungsgemäßen Verbindungen eignen sich deshalb in ausgezeichneter Weise zur selektiven Unkrautbekämpfung in Getreidekulturen.

| Pflanzenart | Wachsstadium | Wuchshöhe (cm) |
|---|---|---|
| TRAE - Triticum aestivum (Sommer) | 13 - 21 | 23 - 25 |
| HOVU - Hordeum vulgare (Sommer) | 13 - 21 | 30 - 32 |
| TRDU - Triticum durum | 21 - 22 | 18 - 20 |
| ALMY - Alopecurus myosuroides | 21 - 22 | 12 - 14 |

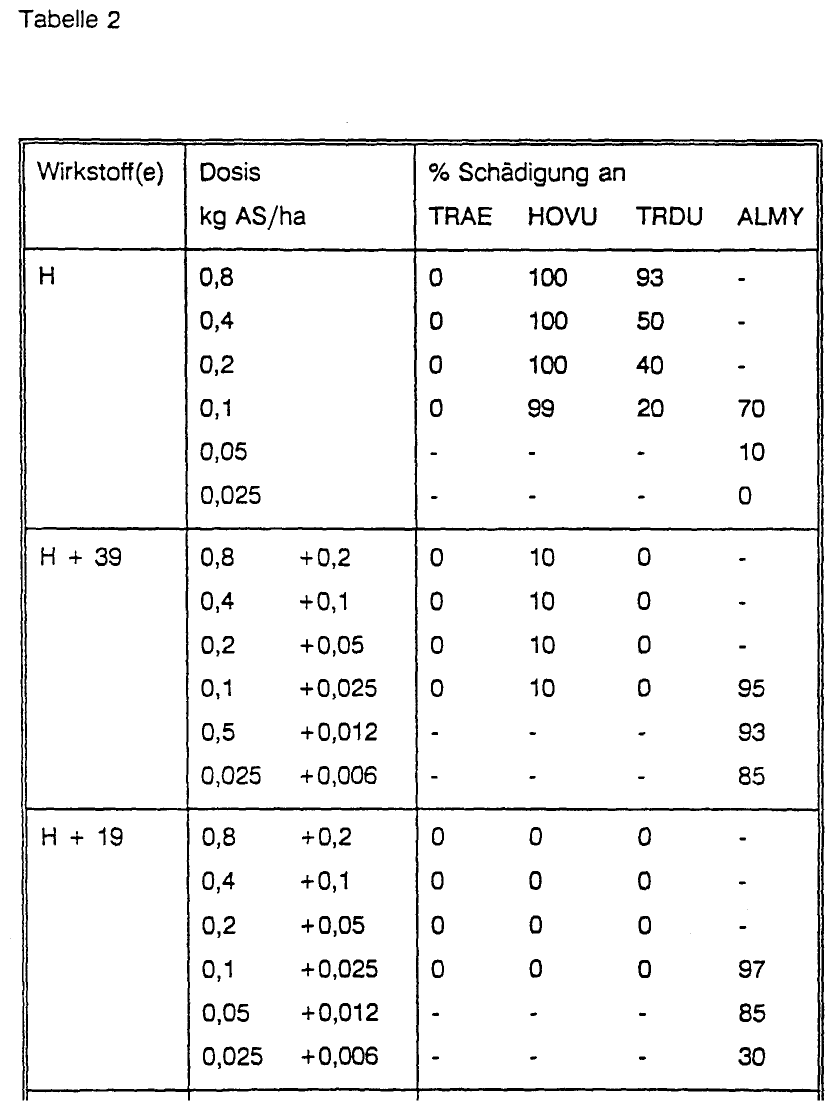

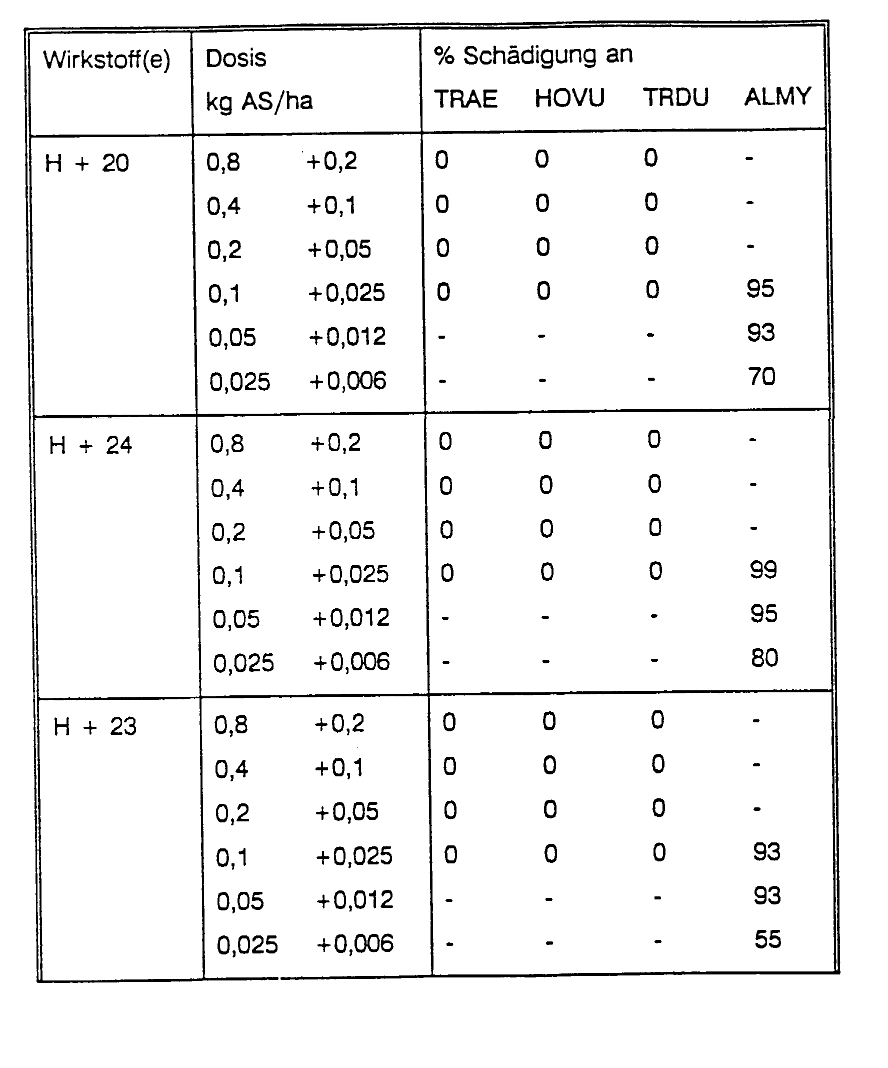

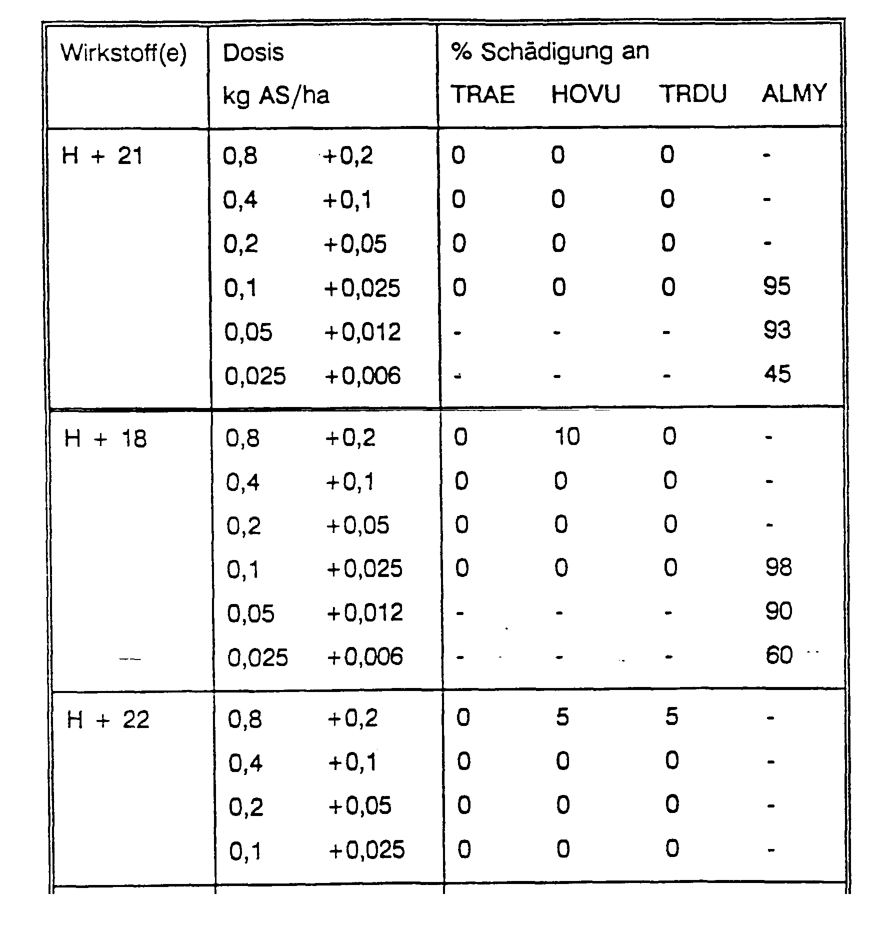

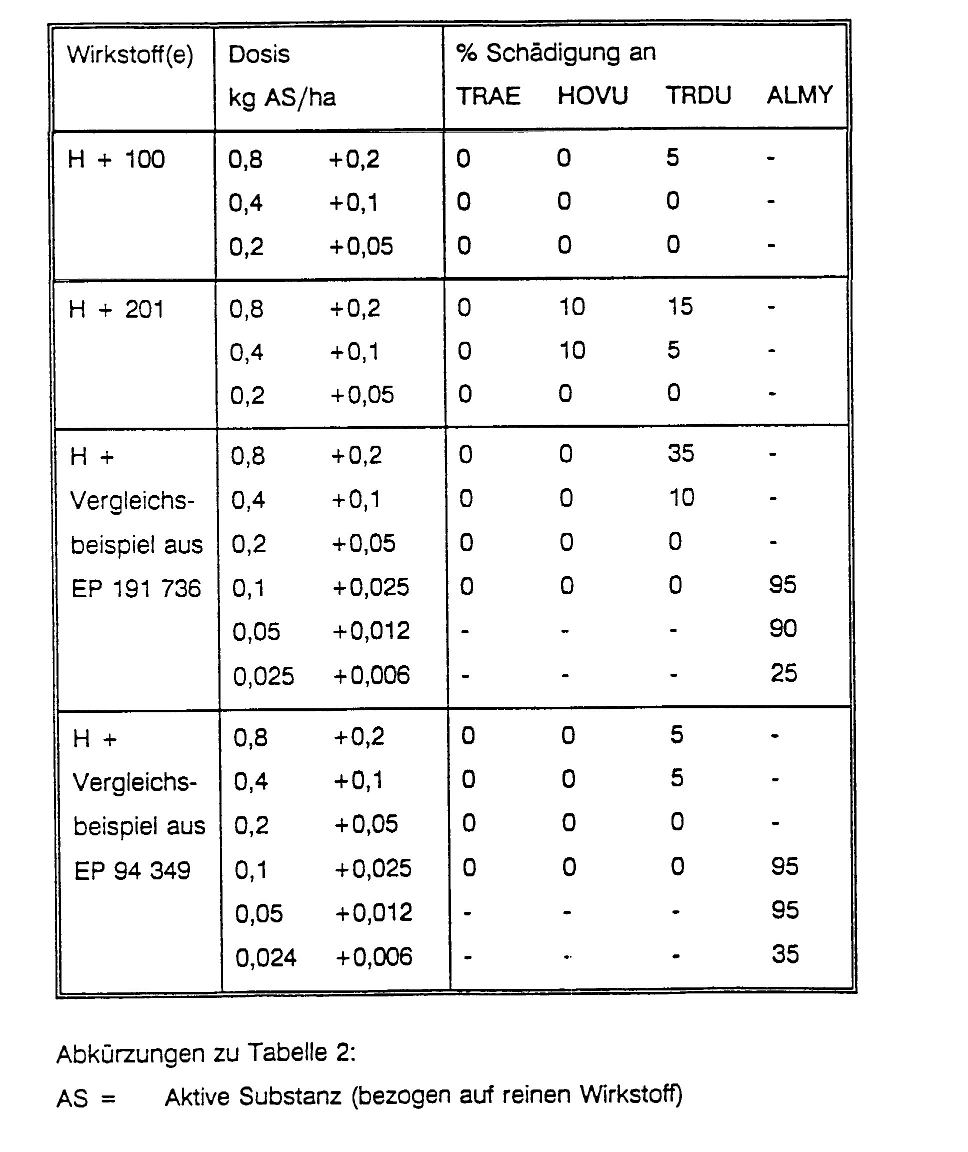

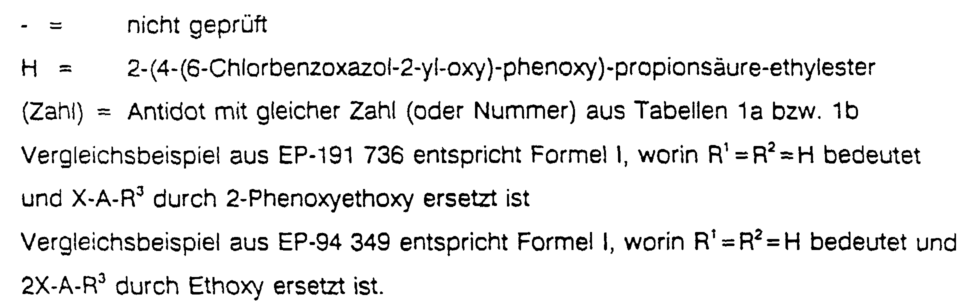

### Beispiel 2

Die Maispflanzen, Unkräuter und Ungräser wurden im Freiland oder im Gewächshaus in Plastiktöpfen bis zum 4- bis 5-Blattstadium herangezogen und nacheinander mit Herbiziden und erfindungsgemäßen Verbindungen der Formel I im Nachauflaufverfahren behandelt. Die Wirkstoffe wurden dabei in Form wäßriger Suspensionen bzw. Emulsionen mit einer Wasseraufwandmenge von umgerechnet 300 l/ha ausgebracht. 4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde. Die Bewertung erfolgte in Prozentwerten im Vergleich zu unbehandelten Kontrollen.

Die Ergebnisse zeigen (siehe z. B. Tabelle 3), daß die erfindungsgemäßen eingesetzten Verbindungen der Formel I starke Herbizidschäden an den Maispflanzen effektiv reduzieren können. Selbst bei starken Überdosierungen der Herbizide werden bei den Kulturpflanzen auftretende schwere Schädigungen deutlich reduziert und geringere Schäden völlig aufgehoben. Mischungen aus Herbiziden und Verbindungen der Formel I eigenen sich deshalb in ausgezeichneter Weise zur selektiven Unkrautbekämpfung in Mais.

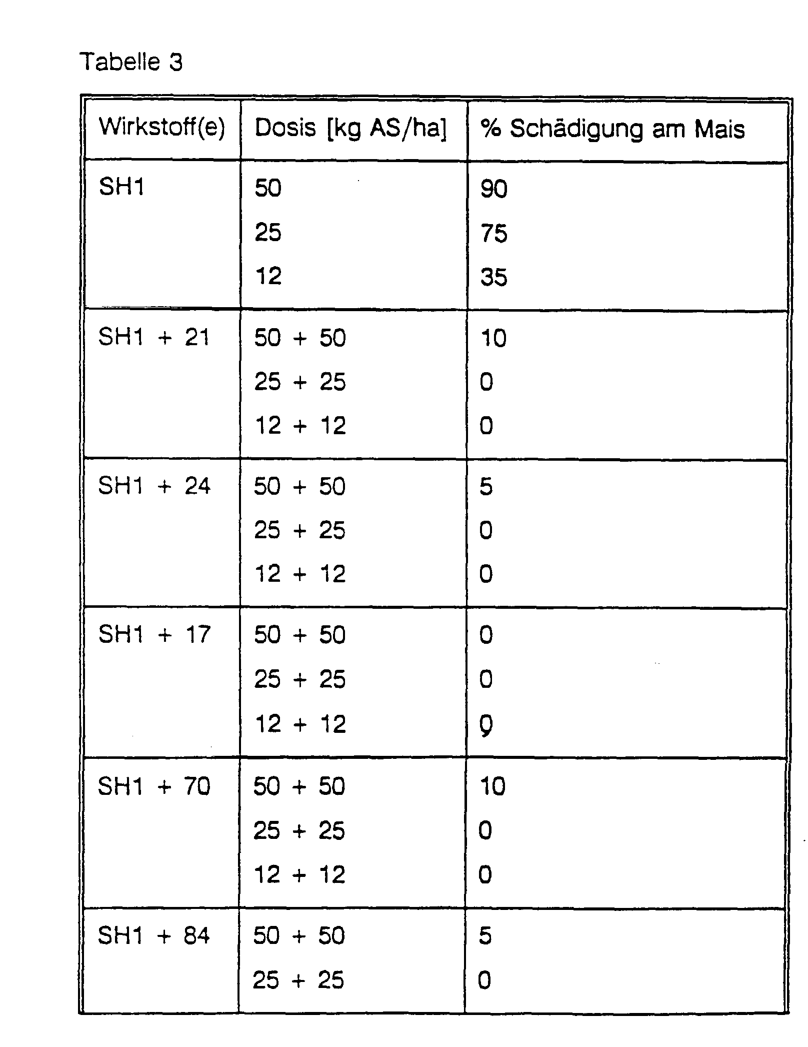

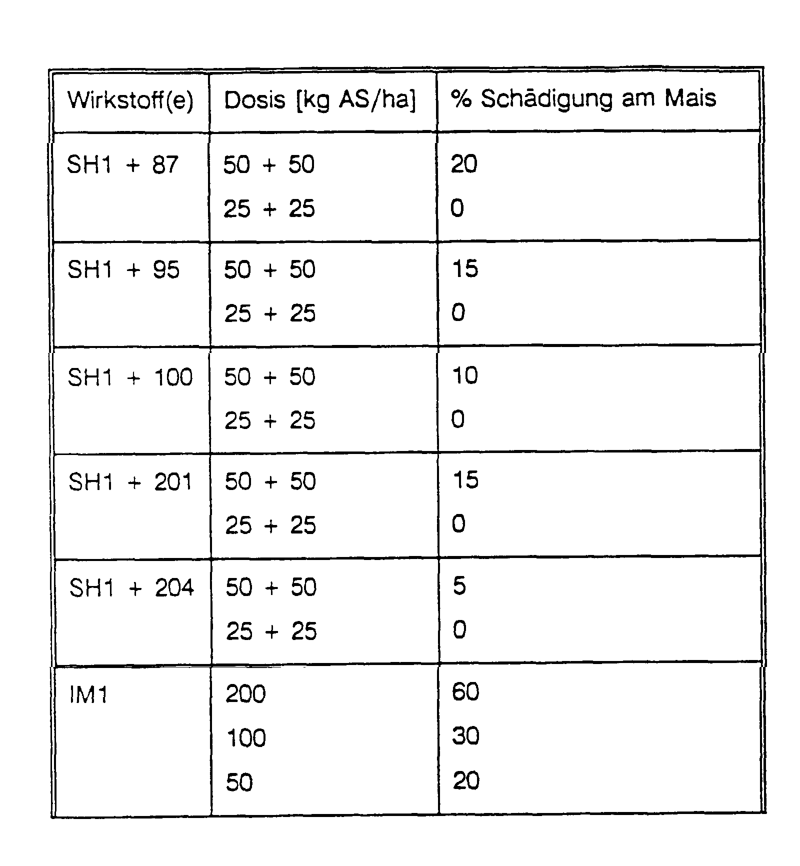

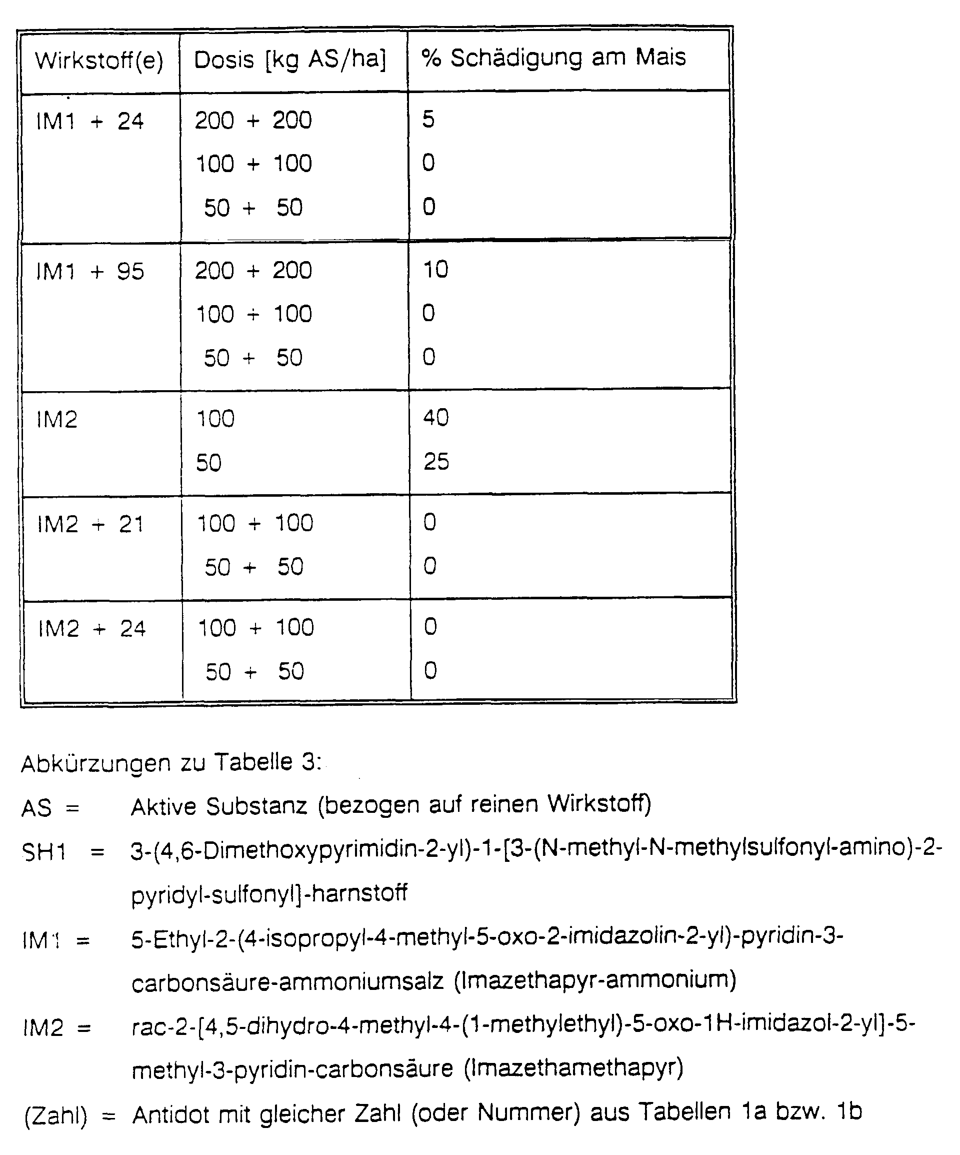

## Patentansprüche

1. Verbindungen der Formel I, 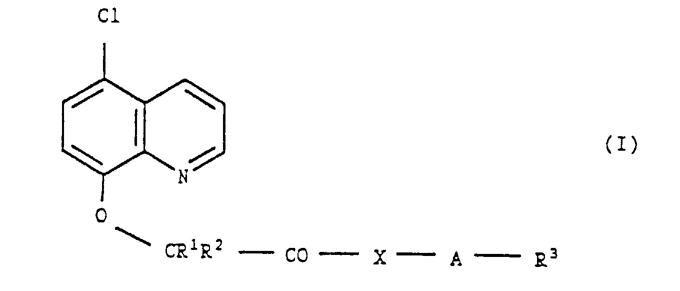 worin
R¹,R² unabhängig voneinander Wasserstoff oder (C₁-C₄)-Alkyl,
X ein Sauerstoff- oder Schwefelatom oder NR⁴, wobei R⁴ Wasserstoff, (C₁-C₆)-Alkyl, (C₁-C₆)-Alkoxy oder gegebenenfalls substituiertes Phenyl bedeutet,
A (C₁-C₆)-Alkylen, (C₄-C₈)-Alkenylen, (C₄-C₈)-Alkinylen, (C₃-C₈)-Cycloalkylen oder (C₃-C₈)-Cycloalkenylen,
R³ (C₃-C₆)-Alkenyloxy, (C₃-C₆)-Alkinyloxy, Phenyl-(C₁-C₄)-alkoxy, worin der Phenylring unsubstituiert oder ein- oder mehrfach durch Reste aus der Gruppe Halogen, Nitro, (C₁-C₄)-Alkyl, (C₁-C₄)-Alkoxy, (C₁-C₄)-Haloalkyl und (C₁-C₄)-Haloalkoxy substituiert ist,
einen Rest der Formel R⁸O-CH(OR⁹)- oder R⁸O-CH(OR⁹)-(CH₂)ₙ-O-, worin n 0, 1 oder 2 bedeutet,
oder einen Alkoxy-Rest der Formel R⁸O-CHR¹⁰-CH(OR⁹)-(C₁-C₄)-alkoxy, (C₁-C₆)-Alkylcarbonyloxy, worin Alkyl unsubstituiert oder durch Halogen, Nitro, gegebenenfalls substituiertes Phenyl oder (C₁-C₄)-Alkoxy substituiert ist,
(C₂-C₆)-Alkenylcarbonyloxy, (C₂-C₆)-Alkinylcarbonyloxy, Phenylcarbonyloxy, wobei Phenyl unsubstituiert oder ein- oder mehrfach durch Reste aus der Gruppe Halogen, Nitro, (C₁-C₄)-Alkyl, (C₁-C₄)-Alkoxy, (C₁-C₄)-Haloalkyl und (C₁-C₄)-Haloalkoxy substituiert ist,
R⁸,R⁹ unabhängig voneinander (C₁-C₄)-Alkyl oder R⁸ und R⁹ zusammen eine geradkettige oder verzweigte (C₁-C₄)-Alkylenbrücke und
R¹⁰ H oder (C₁-C₄)-Alkyl
bedeuten.

2. Verbindungen nach Anspruch 1, dadurch gekennzeichnet, daß
R³ (C₃-C₄)-Alkenyloxy, (C₃-C₄)-Alkinyloxy, Phenyl-(C₁-C₂)-alkoxy, worin der Phenylring unsubstituiert oder ein- oder mehrfach durch Reste aus der Gruppe Halogen, Nitro, (C₁-C₂)-Alkyl, (C₁-C₂)-Alkoxy, (C₁-C₂)-Haloalkyl und (C₁-C₂)-Haloalkoxy substituiert ist,
(C₁-C₄)-Alkylcarbonyloxy, (C₃-C₄)-Alkenylcarbonyloxy, (C₃-C₄)-Alkinylcarbonyloxy,
Phenylcarbonyloxy, wobei Phenyl unsubstituiert oder ein- oder mehrfach durch Reste aus der Gruppe Halogen, Nitro, (C₁-C₄)-Alkyl, (C₁-C₄)-Alkoxy, (C₁-C₄)-Haloalkyl und (C₁-C₄)-Haloalkoxy substituiert ist, oder
einen Rest der Formel -O-CH₂-CH(OR')-CH₂-OR', worin die R' zusammen für die divalente Gruppe CH₂, CHCH₃ oder C(CH₃)₂ stehen,
bedeuten.

3. Verbindungen nach Anspruch 1 oder 2, dadurch gekennzeichnet, daß
R³ (C₃-C₄)-Alkenyloxy, (C₃-C₄)-Alkinyloxy, Benzyloxy,
(C₃-C₄)-Alkenylcarbonyloxy oder (C₃-C₄)-Alkinylcarbonyloxy
bedeuten.

4. Verfahren zur Herstellung von Verbindungen der Formel I nach Anspruch 1, dadurch gekennzeichnet, daß man
a) 5-Chlor-8-hydroxychinolin mit einem Alkancarbonsäurederivat der Formel II,
Y-CR¹R²-CO-X-A-R³ II
worin
Y eine Abgangsgruppe, wie z.B. Chlor, Brom, Methansulfonyl oder Toluolsulfonyl, bedeutet und
R¹,R²,R³ X und A wie bei der genannten Formel I definiert sind, oder
b) 5-Chlorchinolin-8-oxy-alkancarbonsäuren der Formel I, worin -X-A-R³ durch Hydroxy ersetzt ist, mit Alkoholen, Mercaptanen oder Aminen der Formel
H-X-A-R³
wobei X, A und R³ wie bei Formel I definiert sind,
umsetzt.

5. Pflanzenschützende Mittel, dadurch gekennzeichnet, daß sie eine oder mehrere Verbindungen der Formel I nach einem oder mehreren der Ansprüche 1 bis 3 und übliche Formulierungshilfsmittel enthalten.

6. Herbizide Mittel, dadurch gekennzeichnet, daß sie ein oder mehrere Herbizide und ein oder mehrere Verbindungen der Formel I nach einem oder mehreren der Ansprüche 1 bis 3 enthalten.

7. Mittel nach Anspruch 6, dadurch gekennzeichnet, daß die Herbizide aus der Gruppe Carbamate, Thiocarbamate, Halogenacetanilide, substituierte Phenoxy-, Naphthoxy- und Phenoxyphenoxyalkancarbonsäurederivate, Cyclohexandionabkömmlinge, Imidazolinone und Sulfonylharnstoffe sind.

8. Mittel nach Anspruch 6 oder 7, dadurch gekennzeichnet, daß Verbindungen der Formel I (Safener) und Herbizide im Gewichtsverhältnis 1:10 bis 10:1 enthalten sind.

9. Verfahren zum Schutz von Kulturpflanzen vor phytotoxischen Nebenwirkungen von Herbiziden, dadurch gekennzeichnet, daß eine wirksame Menge einer oder mehrerer Verbindungen der Formel I (Safener) nach einem oder mehreren der Ansprüche 1 bis 3 vor, nach oder gleichzeitig mit dem Herbizid auf die Pflanzen, Pflanzensamen oder die Anbaufläche appliziert wird.

10. Verwendung von Verbindungen der Formel I nach einem oder mehreren der Ansprüche 1 bis 3 zur Reduzierung von phytotoxischen Nebenwirkungen von Herbiziden an Kulturpflanzen.

## Claims

1. A compound of the formula I 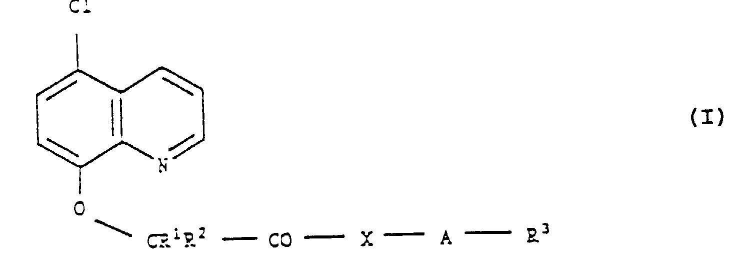 in which
R¹ and R² independently of one another are hydrogen or (C₁-C₄)-alkyl,
X is an oxygen or sulfur atom or NR⁴, in which R⁴ is hydrogen, (C₁-C₆)-alkyl, (C₁-C₆)-alkoxy or optionally substituted phenyl,
A is (C₁-C₆)-alkylene, (C₄-C₈)-alkenylene1 (C₄-C₈)-alkynylene, (C₃-C₈)-cycloalkylene or (C₃-C₈)-cycloalkenylene,
R³ is (C₃-C₆)-alkenyloxy, (C₃-C₆)-alkynyloxy, phenyl-(C₁-C₄)-alkoxy, in which the phenyl ring is unsubstituted or substituted by one or more radicals from the group comprising halogen, nitro, (C₁-C₄)-alkyl, (C₁-C₄)-alkoxy, (C₁-C₄)-haloalkyl and (C₁-C₄)-haloalkoxy,
a radical of the formula R⁸O-CH(OR⁹)- or R⁸O-CH(OR⁹)-(CH₂)ₙ-O-, in which n is 0, 1 or 2,
or an alkoxy radical of the formula R⁸O-CHR¹⁰-CH(OR⁹)-(C₁-C₄)-alkoxy, (C₁-C₆)-alkylcarbonyloxy, in which alkyl is unsubstituted or substituted by halogen, nitro, optionally substituted phenyl or (C₁-C₄)-alkoxy,
(C₂-C₆)-alkenylcarbonyloxy, (C₂-C₆)-alkynylcarbonyloxy,
phenylcarbonyloxy, phenyl being unsubstituted or substituted by one or more radicals from the group comprising halogen, nitro, (C₁-C₄)-alkyl, (C₁-C₄)-alkoxy, (C₁-C₄)-haloalkyl and (C₁-C₄)-haloalkoxy,
R⁸ and R⁹ independently of one another are (C₁-C₄)-alkyl, or R⁸ and R⁹ together are a straight-chain or branched (C₁-C₄)-alkylene bridge and
R¹⁰ is hydrogen or (C₁-C₄)-alkyl.

2. A compound as claimed in claim 1, wherein
R³ is (C₃-C₄)-alkenyloxy, (C₃-C₄)-alkynyloxy, phenyl- (C₁-C₂)-alkoxy, in which the phenyl ring is unsubstituted or substituted by one or more radicals from the group comprising halogen, nitro, (C₁-C₂)-alkyl, (C₁-C₂)-alkoxy, (C₁-C₂)-haloalkyl and (C₁-C₂)-haloalkoxy, (C₁-C₄)-alkylcarbonyloxy, (C₃-C₄)-alkenylcarbonyloxy, (C₃-C₄)-alkynylcarbonyloxy, phenylcarbonyloxy, phenyl being unsubstituted or substituted by one or more radicals from the group comprising halogen, nitro, (C₁-C₄)-alkyl, (C₁-C₄)-alkoxy, (C₁-C₄)-haloalkyl and (C₁-C₄)-haloalkoxy, or
a radical of the formula -O-CH₂-CH(OR')-CH₂-OR', in which the radicals R' together represent the divalent group CH₂, CHCH₃ or C(CH₃)₂.

3. A compound as claimed in claim 1 or 2, wherein
R³ is (C₃-C₄)-alkenyloxy, (C₃-C₄)-alkynyloxy, benzyloxy, (C₃-C₄)-alkenylcarbonyloxy or (C₃-C₄)-alkynylcarbonyloxy.

4. A process for the preparation of a compound of the formula I as claimed in claim 1, which comprises
a) reacting 5-chloro-8-hydroxyquinoline with an alkanecarboxylic acid derivative of the formula II
Y-CR¹R²-CO-X-A-R³ II
in which
Y is a leaving group, such as, for example, chlorine, bromine, methanesulfonyl or toluene-sulfonyl, and
R¹, R², R³, X and A are as defined for the above formula I, or
b) reacting a 5-chloroquinolin-8-oxy-alkanecarboxylic acid of the formula I, in which -X-A-R³ is replaced by hydroxyl, with an alcohol, mercaptan or amine of the formula
H-X-A-R³
in which X, A and R³ are as defined for formula I.

5. A plant protection agent which contains one or more compounds of the formula I as claimed in one or more of claims 1 to 3 and customary formulation auxiliaries.

6. A herbicidal agent which contains one or more herbicides and one or more compounds of the formula I as claimed in one or more of claims 1 to 3.

7. An agent as claimed in claim 6, in which the herbicides are from the group comprising carbamates, thiocarbamates, halogenoacetanilides, substituted phenoxy-, naphthoxy- and phenoxyphenoxyalkanecarboxylic acid derivatives, cyclohexanedione derivatives, imidazolinones and sulfonylureas.

8. An agent as claimed in claim 6 or 7, which contains compounds of the formula I (safeners) and herbicides in a weight ratio of 1:10 to 10:1.

9. A method of protecting crop plants from phytotoxic side effects of herbicides, which comprises applying an effective amount of one or more compounds of the formula I (safeners) as claimed in one or more of claims I to 3 to the plants, plant seeds or cultivation area before, after or at the same time as the herbicide.

10. The use of a compound of the formula I as claimed in one or more of claims 1 to 3 for reducing phytotoxic side effects of herbicides on crop plants.

## Revendications

1. Composés de formule I, 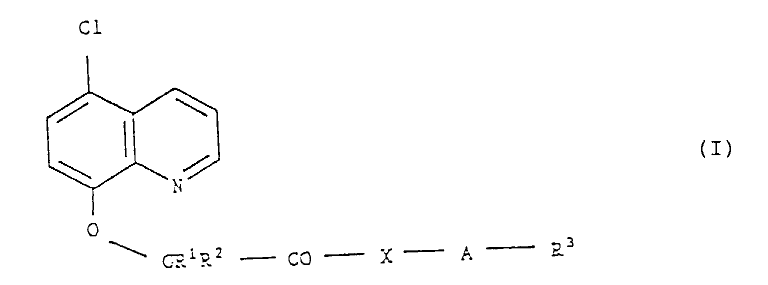 dans laquelle
R¹, R², indépendamment l'un de l'autre, représentent l'hydrogène ou alkyle en C₁-C₄,
X représente un atome d'oxygène ou de soufre, ou NR⁴, R⁴ étant l'hydrogène, alkyle en C₁-C₆, alcoxy en C₁-C₆ ou phényle éventuellement substitué,
A représente alkylène en C₁-C₆, alcénylène en C₄-C₈, alcynylène en C₄-C₈, cycloalkylène en C₃-C₈ ou cycloalcénylène en C₃-C₈,
R³ représente alcényloxy en C₃-C₆, alcynyloxy en C₃-C₆, phényl(alcoxy en C₁-C₄), dans lequel le cycle phényle est non substitué ou substitué une ou plusieurs fois par des radicaux pris dans le groupe comportant halogène, nitro, alkyle en C₁-C₄, alcoxy en C₁-C₄, haloalkyle en C₁-C₄ et haloalcoxy en C₁-C₄,
représente un radical de formule R⁸O-CH(OR⁹)- ou R⁸O-CH(OR⁹)-(CH₂)ₙ-O-, dans laquelle n vaut 0, 1 ou 2,
ou un radical alcoxy de formule R⁸O-CHR¹⁰-CH(OR⁹)-(C₂-C₄) alcoxy,
(alkyle en C₁-C₆)carbonyloxy, où alkyle est non substitué ou substitué par halogène, nitro, phényle éventuellement substitué ou alcoxy en C₁-C₄,
(alcényle en C₂-C₆)carbonyloxy, (alcynyle en C₂-C₆)carbonyloxy, phénylcarbonyloxy, phényle étant non substitué ou substitué une ou plusieurs fois par des radicaux pris dans le groupe comportant halogène, nitro, alkyle en C₁-C₄, alcoxy en C₁-C₄, haloalkyle en C₁-C₄ et haloalcoxy en C₁-C₄,
R⁸, R⁹ indépendamment l'un de l'autre, représentent alkyle en C₁-C₄ ou R⁸ et R⁹ ensemble forment un pont alkylène en C₁-C₄ linéaire ou ramifié et
R¹⁰ représente H ou alkyle en C₁-C₄.

2. Composés selon la revendication 1, caractérisés en ce que
R³ représente (alcényle en C₃-C₄)oxy, (alcynyle en C₃-C₄)oxy, phényl(alcoxy en C₁-C₂), le cycle phényle pouvant être non substitué ou une ou plusieurs fois substitué par des radicaux pris dans le groupe comportant halogène, nitro, alkyle en C₁-C₂, alcoxy en C₁-C₂, haloalkyle en C₁-C₂ et haloalcoxy en C₁-C₂,
représente (alkyle en C₁-C₄)carbonyloxy, (alcényle en C₃-C₄)-carbonyloxy, (alcynyle en C₃-C₄)-carbonyloxy, phénylcarbonyloxy, phényle pouvant être non substitué ou substitué une ou plusieurs fois par des radicaux pris dans le groupe comportant halogènes, nitro, alkyle en C₁-C₄, alcoxy en C₁-C₄, haloalkyle en C₁-C₄ et haloalcoxy en C₁-C₄, ou un radical de formule -O-CH₂-CH(OR')-CH₂-OR', dans laquelle les radicaux R' ensemble représentent le groupe bivalent CH₂, CHCH₃ ou C(CH₃)₂.

3. Composés selon la revendication 1 ou 2, caractérisés en ce que
R³ représente (alcényle en C₃-C₄)oxy, (alcynyle en C₃-C₄)oxy, benzyloxy, (alcényle en C₃-C₄)-carbonyloxy ou (alcynyle en C₃-C₄)carbonyloxy.

4. Procédé pour la préparation de composés de formule I selon la revendication 1, caractérisé en ce que l'on fait réagir
a) la 5-chloro-8-hydroxyquinoléine avec un dérivé d'acide alcanecarboxylique de formule II
Y-CR¹R²-CO-X-A-R³ (II)
dans laquelle
Y représente un groupe éliminable comme par exemple chloro, bromo, méthanesulfonyle ou toluènesulfonyle, et
R¹, R², R³, X et A sont définis dans la formule I citéé ci-dessus, ou
b) les acides 5-chloroquinoléine-8-oxyalcanecarboxyliques de formule I dans laquelle -X-A-R³ est remplacé par hydroxy, avec des alcools, des mercaptans ou des amines de formule
H-X-A-R³
X, A et R³ ayant les définitions de la formule I.

5. Agents phytoprotecteurs caractérisés en ce qu'ils contiennent un ou plusieurs composés de formule I selon une ou plusieurs des revendications 1 à 3 et des adjuvants de formulation usuels.

6. Agents phytoprotecteurs caractérisés en ce qu'ils contiennent un ou plusieurs herbicides et un ou plusieurs composés de formule I selon une ou plusieurs des revendications 1 à 3.

7. Agents selon la revendication 6, caractérisés en ce que les herbicides pris dans le groupe comportant les carbamates, les thiocarbamates, les halogénoacétanilides, les dérivés substitués de l'acide phénoxy-, naphtoxy- et phénoxyphénoxyalcanecarboxylique, les dérivés de cyclohexanedione, les imidazolinones et les sulfonylurées.

8. Agents selon la revendication 6 ou 7, caractérisés en ce que les composés de formule I (agent protecteur) et les herbicides sont contenus dans un rapport pondéral de 1:10 à 10:1.

9. Procédé pour la protection des plantes de culture contre les effets phytotoxiques indésirables des herbicides, caractérisé en ce que l'on applique une quantité efficace d'un ou plusieurs composés de formule I (agent protecteur) selon une ou plusieurs des revendications 1 à 3, avant, après ou conjointement avec l'herbicide sur les plantes, la semence ou les surfaces cultivables.

10. Utilisation des composés de formule I selon une ou plusieurs des revendications 1 à 3, pour réduire les effets phytotoxiques indésirables des herbicides sur les plantes de culture.
